# EUROPEAN PATENT SPECIFICATION

(11) **EP 2 204 152 B1**
(45) Date of publication and mention of the grant of the patent: **18.12.2013**
(21) Application number: 08790712.7
(22) Date of filing: 27.06.2008
(51) Int. Cl.: A61F 13/49, A61F 13/511

(54) **DISPOSABLE DIAPER**
EINWEGWINDEL
COUCHE JETABLE

(30) Priority: 29.06.2007 JP 2007171434; 29.06.2007 JP 2007171435; 31.07.2007 JP 2007200146
(43) Date of publication of application: 07.07.2010
(73) Proprietor: Daio Paper Corporation, Ehime 799-0492 (JP)
(72) Inventor: MURAI, Kosuke, Shikokuchuo-shi Ehime 799-0431 (JP)
(74) Representative: Tollett, Ian
(86) International application number: PCT/JP2008/061747
(87) International publication number: WO 2009/005006

(56) References cited:
- EP-A1- 1 803 430
- EP-A1- 1 908 441
- WO-A1-00/24351
- JP-A- 02 055 058
- JP-A- 2002 177 327
- JP-A- 2002 291 796

## Description

### Technical Field

The present invention relates to disposable diapers.

### Background Art

In general, a newborn infant firstly gets out watery stool and then comes to produce harder stool in three months or so. After start of baby food, the infant's stool becomes more solid. Therefore, there have been proposed various techniques associated with disposable diapers for infants in consideration of absorption of watery and soft stool (hereinafter, referred to as watery stool and the like) (see Patent Documents 1 and 2, for example). Among them, a technique of forming holes for letting stool pass through a liquid pervious top sheet constituting a surface of a diaper touching the body of a wearer, has been proven to be effective in absorption of watery stool and the like.
Patent Document 1: JP 2005-246811 A
Patent Document 2: JP 2812340 B
Patent Document 3: JP 2006-149457 A
Patent Document 4: JP 2006-239290 A

### Disclosure of the Invention

### Technical Problems to be Solved

Despite being effective in absorbing watery stool and the like, however, such a conventional diaper with permeation holes on the top sheet has a problem that, when the wearer keeps on the diaper for a certain period of time after excretion, liquid or solid contents of excrement are prone to attach to the tops and their surrounding sections of the right and left gluteal regions of the wearer.
Accordingly, a main object of the present invention is to solve the foregoing problem without deteriorating the diaper in a capability of absorbing watery stool and the like.

### Means to Solve the Problems

### <Invention according to Claim 1>

A disposable diaper, comprising an absorbent part which has a liquid pervious sheet constituting a surface facing the body of a wearer, a liquid impervious sheet located on an external side and an absorbent element interposed between the liquid pervious sheet and the liquid impervious sheet and which extends from an upper edge of a ventral side through a crotch portion to an upper edge of the back side along a center in the width direction, wherein
the top sheet has a widthwise central region extending from front to back ends through a portion corresponding to an intergluteal cleft; left- and right-side intermediate regions that are positioned on left and right sides of the widthwise central region and extend from front to back ends through portions corresponding to tops of left and right gluteal regions and, respectively; and left - and right -side regions that extend from front to back ends along a left side of the left-side intermediate region and a right side of the right-side intermediate region, respectively,
a large number of permeation holes are formed at predetermined intervals in the widthwise central region at least at an entire widthwise section in a front-back direction corresponding to the intergluteal cleft; in the left-side region at least at an entire widthwise section in the front-back direction corresponding to a section located on a left side of the top of the left gluteal region in the width direction; and in the right-side region at least at an entire widthwise section in the front-back direction corresponding to a section located on a right side of the top of the right gluteal region in the width direction, and no permeation hole is formed in the left-side intermediate region at least at a section corresponding to the top of the left gluteal region and in the right-side intermediate region at least at a section corresponding to the top of the right gluteal region.

### (Effect and operation)

The inventor's earnest studies on the foregoing problem have produced a finding as stated below. Specifically, since a disposable diaper is under stronger pressure on sections corresponding to the right and left gluteal regions of the wearer, when permeation holes are uniformly formed in all the sections of the top sheet, including the sections corresponding to the right and left gluteal regions as in conventional diapers, an excreted matter having passed through the top sheet flows back to the surface of the top sheet under pressure from the gluteal region and attaches to the tops and their surrounding parts of the right and left gluteal regions. The present invention has been devised on the basis of the foregoing finding.
The section of the diaper corresponding to an intergluteal cleft does not touch the body of the wearer or touch the body of the wearer under slight pressure, and the section is also located at a position of excretion of stool. Accordingly, by forming a large number of permeation holes at least in the foregoing section (corresponding to the intergluteal cleft in the widthwise central region), it is possible to quickly capture excreted watery stool and the like into the diaper. In addition, the sections of the diaper corresponding to the right and left gluteal regions (bulges) are under strong pressure, but no permeation hole is formed in those sections (corresponding to the right and left gluteal regions in the right- and left-side intermediate regions), whereby an excreted matter is less prone to flow back and attach to the gluteal region unlike conventional diapers. In this case, an excreted matter having not been captured in the widthwise central region and having moved to the both sides of the widthwise central region, is not captured also in the right- or left-side intermediate region and is more prone to pass over the right and left gluteal regions and move outward in the width direction. However, by forming permeation holes on the further outsides of the right-and left-side intermediate regions in the width direction (in the sections corresponding to a section in the left-side region located on the left side of the left gluteal region in the width direction and corresponding to a section in the right-side region located on the right side of the right gluteal region in the width direction), it is possible to effectively capture an excreted matter passing over the gluteal region into the diaper. In addition, needless to say, the permeation holes in the present invention are holes penetrating through the top sheet, not cavities.

### <Invention according to Claim 2>

The disposable diaper according to Claim 1, wherein
a width of the right- and left-side regions is smaller than a width of the widthwise central region.

### (Effect and operation)

It is preferred that the width of the right- and left-side regions is smaller than the width of the central region as described in this claim, since a decreased amount of excretion flows back from the permeation holes in the right- and left-side region and attaches to the gluteal region of the wearer when the wearer lies down on his/her side.

### <Invention according to Claim 3>

The disposable diaper according to Claim 1, wherein
the top sheet is formed by a nonwoven fabric with a fineness of raw fibers of 1.0 to 3.0 dtex and a fiber basis weight of 10 to 30 g/m²,
the permeation holes are arranged in such a pattern that permeation hole lines with the permeation holes aligned at predetermined intervals in the front-back direction, are provided at predetermined intervals in the width direction,
an opening area of the permeation hole is 0.8 to 180 mm², a widthwise interval between the permeation hole lines is 1 to 5 mm, and a front-back interval between the permeation holes in the permeation hole lines is 1 to 10 mm, in each of the widthwise central region and the right- and left-side regions.

### (Effect and operation)

The top sheet uses preferably a nonwoven fabric as described in this claim from the viewpoints of texture, strength and the like. In this case, it is meaningless to provide the permeation holes if the opening areas of the permeation holes are not larger than inter-fiber gaps. In addition, if the opening areas of the permeation holes are simply larger than the inter-fiber gaps, the diaper may be insufficient in a capability of capturing watery stool and the like. Further, if the permeation holes have large opening areas but are sparsely arranged in small numbers, the diaper is decreased in a capability of capturing watery stool and the like. Accordingly, it is preferred that the opening area and arrangement interval of the permeation holes fall within the ranges described in this claim.

### <Invention according to Claim 4>

The disposable diaper according to Claim 3, wherein
the permeation hole has an elongated opening shape in which a front-back length is 4 to 15 mm and a widthwise length is smaller than the front-back length.

### (Effect and operation)

The opening shape of the permeation hole can be decided as appropriate, and is preferably the elongated shape as described in this claim, which can capture easily an excreted matter moving in the width direction.

### <Invention according to Claim 5>

The disposable diaper according to Claim 1, wherein
the permeation holes in the right- and left-side regions have each a widthwise opening length of 2 to 7 mm, and the permeation holes in the widthwise central region have each a widthwise opening length of 4 to 10 mm, and the widthwise length of each of the permeation holes in the right- and left-side regions is smaller than the widthwise length of each of the permeation holes in the widthwise central region.

### (Effect and operation)

When the widthwise opening length of the permeation holes in the right- and left-side regions is smaller than the widthwise opening length of those in the widthwise central region as described in this claim, a total area of the permeation holes touching the gluteal region of the wearer becomes small when the wearer lies down on his/her side. This provides an advantage that a decreased amount of excretion flows back from the permeation holes and attaches to the gluteal region.

### <Invention according to Claim 6>

The disposable diaper according to Claim 1, wherein
the permeation holes in the widthwise central region have each an opening area of 5 to 40 mm², the permeation holes in the right- and left-side regions have each an opening area of 10 to 50 mm², and the opening area of each of the permeation holes in the right- and left-side regions is larger than the opening area of each of the permeation holes in the widthwise central region.

### (Effect and operation)

Stool with a high liquid content, such as watery stool, as moving outward from the widthwise central region, tends to become higher in solid content proportion because a liquid content thereof is gradually absorbed. Accordingly, by forming the permeation holes with larger opening areas in the right- and left-side regions as described in this claim, it is possible to effectively capture stool with a high solid content having reached the right- and left-side regions into the diaper.

### <Invention according to Claim 7>

The disposable diaper according to Claim 1, wherein
the top sheet has left- and right-side side edge regions extending from front to back ends along a left side of the left-side region and a right side of the right-side region, respectively,
no permeation hole is formed in the left- and right-side side edge regions, and
a large number of welded points are provided at predetermined intervals to fix the top sheet to a member on an under side thereof, in a first section of ± 10 mm or less in the width direction from both side edges of the permeation hole group in the width direction in the widthwise central region; in a second section of ± 10 mm or less in the width direction from both side edges of the permeation hole group in the left-side region; and in a third section of ± 10 mm or less in the width direction from both side edges of the permeation hole group in the right-side region, the top sheet is fixed to the member on the under side thereof without using an adhesive, and the widthwise central region and the left- and right-side regions are not fixed to the member on the under side excluded from a scope of the first to third sections.

### (Effect and operation)

If the top sheet has permeation holes, there is a problem that the permeation holes may be broken by widthwise contraction of the top sheet, in particular at the crotch portion, thereby deteriorating the diaper in a capability of letting watery stool and the like pass through the permeation holes. A possible solution for this problem is to weld and fix the top sheet in spots to the member on the under side thereof as described in Patent Documents 3 and 4. However, this solution can prevent breakage of the permeation holes but is disadvantageous in that the diaper's capability of letting watery stool and the like pass through the permeation holes becomes low beyond expectation. Accordingly, there is a demand for a solution that makes it possible to prevent breakage of the permeation holes and provide a sufficient capability of letting a solid content pass through.
Then, the inventor's earnest studies on the foregoing problem have produced a finding as described below. Specifically, if the top sheet is fixed by welding in such a manner that permeation hole groups are closely arranged at the fixed sections, clearances are less prone to be created between the top sheet and the member on the under side thereof. As a result, watery stool is less prone to be captured into the top sheet on the under side thereof.
A disposable diaper is generally under stronger pressure at sections corresponding to the tops of the right and left gluteal regions. Accordingly, if permeation holes are uniformly provided at all the sections of the top sheet including the sections corresponding to the tops of the right and left gluteal regions as in conventional diapers, an excreted matter having passed through the top sheet flows back into the surface of the top sheet under a pressure from the tops of the right and left gluteal regions and attaches to the tops and their surrounding parts of the right and left gluteal region. Therefore, it is desired that no permeation hole is formed at the foregoing sections.
More specifically, the section of the diaper corresponding to the intergluteal cleft does not touch the body of the wearer or touch the body of the wearer under slight pressure, and the section is also located at a position of excretion of stool. Accordingly, by forming a large number of permeation holes at least in the foregoing section (corresponding to the intergluteal cleft in the widthwise central region), it is possible to quickly capture excreted watery stool and the like into the diaper. In addition, the sections of the diaper corresponding to the right and left gluteal regions (bulges) are under strong pressure, but no permeation hole is formed in those sections (corresponding to the tops of the right and left gluteal regions in the right- and left-side intermediate regions), whereby an excreted matter is less prone to flow back and attach to the gluteal region unlike conventional diapers. In this case, an excreted matter having not been captured in the widthwise central region and having moved to the both sides of the widthwise central region, is not captured also in the right- or left-side intermediate region and is more prone to pass over the right and left gluteal regions and move outward in the width direction. However, by forming permeation holes on the further outsides of the right- and left-side intermediate regions in the width direction (in the sections corresponding to a section in the left-side region located on the left side of the top of the left gluteal region in the width direction and a section in the right-side region located on the right side of the top of the right gluteal region in the width direction), it is possible to effectively capture an excreted matter passing over the tops of the left and right gluteal regions into the diaper.
In addition, the left-side side edge section of the left-side region and the right-side side edge section of the right-side region are used for fixation of the top sheet, and therefore those sections are not preferably provided with permeation holes.
The invention of this claim employs an arrangement of the permeation hole group with such an advantage as described above. In the invention of this claim, welded points are provided for fixation in sections of ± 10 mm or less in the width direction from the both side edges of the permeation hole groups in the width direction, and outside the foregoing sections, the top sheet is not fixed in the permeation hole groups and therefore no welded points are provided and no adhesive is used. Accordingly, the sections with the permeation hole groups are defined in width by the welded points on the both sides, thereby to suppress breakage of the permeation holes. In addition, in sections between the welded points, clearances are created between the sections with the permeation holes and the member on the under side, which allows watery stool and the like to be easily captured through the permeation holes. Here, the first to third sections are each set at ± 10 mm in the width direction from the reference side edges, making an allowance for inevitable displacement of the top sheet at a time of formation of the welded points. In this case, the welded points and the permeation holes may overlap, but needless to say, in a preferred embodiment, the welded points and the permeation holes do not overlap in the width direction. In addition, needless to say, the permeation holes in the present invention are holes penetrating through the top sheet, not cavities.

### <Invention according to Claim 8>

The disposable diaper according to Claim 7, wherein
the permeation holes are formed by providing front-back slits in the top sheet at permeation hole formation positions and stretching the top sheet in the width direction to thereby extend the slits, and the top sheet is adhered, while the slits are thus extended, to a member on the under side of the top sheet, at regions in vicinities of the both side edges of the permeation hole groups in the width direction.

### (Effect and operation)

By forming the permeation holes in such a manner as described above, the soft and high-liquid pervious top sheet can be preferably obtained. In addition, by adhering the top sheet in an extended state to the member on the under side of the top sheet at the regions in the vicinities of the both side edges of the permeation hole groups in the width direction, it is possible to fix the top sheet firmly while maintaining the openings of the permeation holes in a preferred manner.

### <Invention according to Claim 9>

The disposable diaper according to Claim 7, wherein
the top sheet is formed by a nonwoven fabric with a fineness of raw fibers of 1.0 to 3.0 dtex and a fiber basis weight of 10 to 30 g/m²,
the permeation holes are arranged in such a pattern that permeation hole lines with the permeation holes aligned at predetermined intervals in the front-back direction, are provided at predetermined intervals in the width direction,
the welded points are arranged in such a pattern that welded point lines with the welded points aligned at predetermined intervals in the front-back direction, are provided at predetermined intervals in the width direction,
an opening area of the permeation hole is 0.8 to 180 mm², a widthwise interval between the permeation hole lines is 1 to 5 mm, and a front-back interval between the permeation holes in the permeation hole lines is 1 to 10 mm, in each of the widthwise central region and the right- and left-side regions, and
an area of the welded point is 0.7 to 40 mm², a widthwise interval between the welded point lines is 2 to 15 mm, and a front-back interval between the welded points in the welded point lines is 5 to 30 mm.

### (Effect and operation)

The top sheet uses preferably a nonwoven fabric as described in this claim from the viewpoints of texture, strength and the like. In this case, it is meaningless to provide the permeation holes if the opening areas of the permeation holes are not larger than inter-fiber gaps. In addition, if the opening areas of the permeation holes are simply larger than the inter-fiber gaps, the diaper may be insufficient in a capability of capturing watery stool and the like. Further, if the permeation holes have large opening areas but are sparsely arranged in small numbers, the diaper is decreased in a capability of capturing watery stool and the like. Accordingly, it is preferred that the opening area and arrangement interval of the permeation holes fall within the ranges described in this claim. In addition, when the areas and arrangement intervals of the welded points fall within the ranges specified in this claim with respect to the foregoing areas and arrangement intervals of the permeation holes, even if the disposable diaper becomes bent and narrow at the crotch portion in the width direction, it is possible to maintain the openings of the permeation holes in a stable manner and thus capture watery stool and the like into the diaper at any time in a reliable manner.

### <Invention according to Claim 10>

The disposable diaper according to Claim 7, wherein
in the first section, occupied areas of the welded points on a left side of the permeation hole group in the width direction in the widthwise central region are larger with distant from the permeation hole group in the widthwise central region toward the left in the width direction; in the first section, occupied areas of the welded points on a right side of the permeation hole group in the width direction in the widthwise central region are larger with distant from the permeation hole group in the widthwise central region toward the right in the width direction,
in the second section, occupied areas of the welded points on a left side of the permeation hole group in the width direction in the left-side region are larger with distant from the permeation hole group in the left-side region toward the left in the width direction; and in the second section, occupied areas of the welded points on a right side of the permeation hole group in the width direction in the left-side region are larger with distant from the permeation hole group in the left-side region toward the right in the width direction, and
in the third section, occupied areas of the welded points on a left side of the permeation hole group in the width direction in the right-side region are larger with distant from the permeation hole group in the right-side region toward the left in the width direction; and in the third section, occupied areas of the welded points on a right side of the permeation hole group in the width direction in the right-side region are larger with distant from the permeation hole group in the right-side region toward the right in the width direction.

### (Effect and operation)

By configuring the occupied areas of the welded points so as to be larger with distant in the width direction from the side edges of the reference permeation hole groups as stated above, the welded points and the permeation holes are less prone to overlap or the welded points and the permeation holes overlap only by limited areas even if the top sheet is displaced in the width direction at a welding process. In addition, if the top sheet has some unfixed sections, the top sheet is inevitably decreased in fixation strength. However, by configuring the occupied areas of the welded points so as to be larger with distance from the side edges of the reference permeation hole groups, the welded points increases the top sheet in fixation strength with distance from the side edges of the permeation hole groups, which compensates for decrease in fixation strength of the top sheet to some extent at the unfixed sections.

### <Invention according to Claim 11>

The disposable diaper according to Claim 7, wherein
out of the welded points in the first section, the welded points on the left side of the permeation hole group in the width direction in the widthwise central region have each a shape that is larger in a front-back length with distant from the permeation hole group in the widthwise central region toward the left in the width direction; and out of the welded points in the first section, the welded points on the right side of the permeation hole group in the width direction in the widthwise central region have each a shape that is larger in a front-back length with distant from the permeation hole group in the widthwise central region toward the right in the width direction,
out of the welded points in the second section, the welded points on the left side of the permeation hole group in the width direction in the left-side region have each a shape that is larger in a front-back length with distant from the permeation hole group in the left-side region toward the left in the width direction; and out of the welded points in the second section, the welded points on the right side of the permeation hole group in the width direction in the left-side region have each a shape that is larger in a front-back length with distant from the permeation hole group in the left-side region toward the right in the width direction, and
out of the welded points in the third section, the welded points on the left side of the permeation hole group in the width direction in the right-side region have each a shape that is larger in a front-back length with distant from the permeation hole group in the right-side region toward the left in the width direction; and out of the welded points in the third section, the welded points on the right side of the permeation hole group in the width direction in the right-side region have each a shape that is larger in a front-back length with distant from the permeation hole group in the right-side region toward the right in the width direction.

### (Effect and operation)

By configuring the shape of the welded points so as to be larger in the front-back length with distant from the side edges of the reference permeation hole groups in the width direction, it is possible to provide the same advantage as that of the invention of Claim 3.

### <Invention according to Claim 12>

The disposable diaper according to Claim 7, wherein
a large number of intermediate region welded points are provided at predetermined intervals to fix the top sheet to a member on the under side thereof, in the left- and right-side intermediate regions excluded from a scope of the first to third sections, and
areas of the intermediate region welded points are larger than the areas of the welded points in the first to third sections.

### (Effect and operation)

By configuring the areas of the intermediate region welded points so as to be larger than the areas of the welded points in the first to third sections as stated above, it is possible to improve the top sheet in fixation strength. In addition, by welding the top sheet in larger areas at the sections under pressure from the tops of the right and left gluteal regions, it is possible to further reduce backflow of excretion at the foregoing sections.

### <Invention according to Claim 13>

The disposable diaper according to Claim 1, further comprising:
an interlayer sheet between the top sheet and the absorbent element so as to contact the under side of the top sheet, wherein
the interlayer sheet has wavelike wrinkles formed by fixing elastic members in a stretched state to a base material sheet, and then contracting the base material sheet by contraction of the elastic members.

### (Effect and operation)

In conventional disposable diapers, watery stool and the like is less prone to pass through the top sheet with no clearance on the under side of the top sheet, and therefore watery stool and the like may spread over the top sheet surface and soil the skin of the wearer in a wide area. Particularly, in conventional disposable diapers with permeation holes in the top sheet, the permeation holes are not fully effective due to absence of clearance on the under side of the permeation holes. Therefore, there is a demand for a diaper that is improved in a capability of letting watery stool and the like pass through, thereby to prevent watery stool and the like from spreading over the top sheet surface and reduce soiling of the skin of the wearer.
In conventional diapers, an interlayer sheet (called also second sheet) is interposed between the top sheet and the absorbent element so that an excreted matter having passed through the top sheet can move to the absorbent body, thereby preventing backflow of the excretion. Conventionally, the interlayer sheet uses a high-bulk nonwoven fabric formed by thick fibers, and absorbs in wide inter-fiber gaps watery stool and the like having passed through the top sheet. In this case, however, the permeation holes are almost filled by the interlayer sheet even with wide inter-fiber gaps.
Meanwhile, in the invention of this claim, the interlayer sheet is provided with wavelike wrinkles on the surface thereof such that linearly continuous spaces are formed between the concave parts of the wrinkles and the top sheet. Accordingly, watery stool and the like having passed through the top sheet are received and spread in the spaces, which leads to increase in a possible permeation amount of watery stool and the like as compared with conventional cases. As a result, watery stool and the like are less prone to spread over the top sheet surface, thereby decreasing soiling of the skin of the wearer. In addition, since the interlayer sheet are wrinkled by contraction forces of the elastic members, even if the interlayer sheet is temporarily smoothed out by external pressure, the interlayer sheet is forcedly returned to the original state upon removal of the external pressure, thereby to ensure the above-mentioned spaces between the interlayer sheet and the top sheet. Accordingly, the above-mentioned effect of improving liquid perviousness can be produced in a sustainable and reliable manner.

### <Invention according to Claim 14>

The disposable diaper according to Claim 13, wherein
the interlayer sheet is formed by sticking together two nonwoven fabrics with a fineness of 2.0 to 5.0 dtex, a fiber basis weight of 10 to 30 g/m², and a thickness of 0.1 to 4.0 mm; fixing a plurality of elongated resilient and elastic members with a fineness of 150 to 1,240 dtex in parallel in a 100 to 300% stretched state and at intervals of 3 to 15 mm between the foregoing nonwoven fabrics; and contracting the base material sheet by contraction of the elastic members, thereby to form wavelike wrinkles on the base material sheet surface in a direction crossing with the elastic members.

### (Effect and operation)

With such a structure as stated above, the above-mentioned effect of the interlayer sheet can be further enhanced. In addition, by using such nonwoven fabrics as base materials, a solid content in watery stool and the like is filtered by inter-fiber gaps in the nonwoven fabrics. Accordingly, only a liquid content of the same reaches the absorbent element and is absorbed in the same, which does not deteriorate the absorbent element in absorption performance.

### <Invention according to Claim 15>

The disposable diaper according to Claim 14, wherein
the wrinkles are formed in a diaper width direction.

### (Effect and operation)

When the direction of the wrinkles is aligned to the diaper width direction, watery stool and the like is less prone to spread in the front-back direction, thereby preventing effectively the urination parts and others of the wearer from being soiled with the stool.

### <Invention according to Claim 16>

A disposable diaper according to claim 1, wherein
an interlayer sheet is interposed between the top sheet and the absorbent element so as to contact the under side of the top sheet,
the interlayer sheet is formed by fixing elastic members in a stretched state to a base material sheet and contracting the base material sheet by contraction of the elastic members, thereby to form wavelike wrinkles on the base material sheet surface, and the wrinkles are at least partly located on the under side of the permeation holes in the top sheet.

### (Effect and operation)

In the invention of this claim, watery stool and the like having passed through the permeation holes in the top sheet, are received and spread in linearly continuous spaces between the concave parts of the wrinkles and the top sheet. Accordingly, the permeation holes fully perform a permeating function. In addition, needless to say, the permeation holes in the present invention are holes penetrating through the top sheet, not cavities.

### <Invention according to Claim 17>

The disposable diaper according to Claim 16, wherein
the interlayer sheet is formed by sticking together two nonwoven fabrics with a fineness of 2.0 to 5.0 dtex, a fiber basis weight of 10 to 30 g/m², and a thickness of 0.1 to 4.0 mm; fixing a plurality of elongated resilient and elastic members with a fineness of 150 to 1,240 dtex in parallel in a 100 to 300% stretched state and at intervals of 3 to 15 mm between the foregoing nonwoven fabrics; and contracting the base material sheet by contraction of the elastic members, thereby to form wavelike wrinkles on the base material sheet surface in a direction crossing with the elastic members.

### (Effect and operation)

With such a structure as stated above, the above-mentioned effect of the interlayer sheet can be further enhanced. In addition, by using such nonwoven fabrics as base materials, a solid content in watery stool and the like is filtered by inter-fiber gaps in the nonwoven fabrics. Accordingly, only a liquid content of the same reaches the absorbent element and is absorbed in the same, which does not deteriorate the absorbent element in absorption performance.

### <Invention according to Claim 18>

The disposable diaper according to Claim 17, wherein
the wrinkles are formed in a diaper width direction.

### (Effect and operation)

When the direction of the wrinkles is aligned to the diaper width direction, watery stool and the like is less prone to spread in the front-back direction, thereby preventing effectively the urination parts and others of the wearer from being soiled with the stool.

### Advantage of the Invention

As described above, according to the present invention, it is possible to effectively suppress attachment of back-flow excretion to the gluteal region of the wearer without deteriorating the diaper in a capability of absorbing watery stool and the like.

### Best Mode for Carrying Out the Invention

As disposable diapers for infants who produce watery or soft stool, tape-type diapers are widely used and therefore the following description of an embodiment of the present invention is provided with a tape-type diaper as an example. However, needless to say, the present invention is also applicable to other types of disposable diapers such as underpants-type disposable diapers. In addition, the present invention can be naturally applied to also disposable diapers for adults as well.

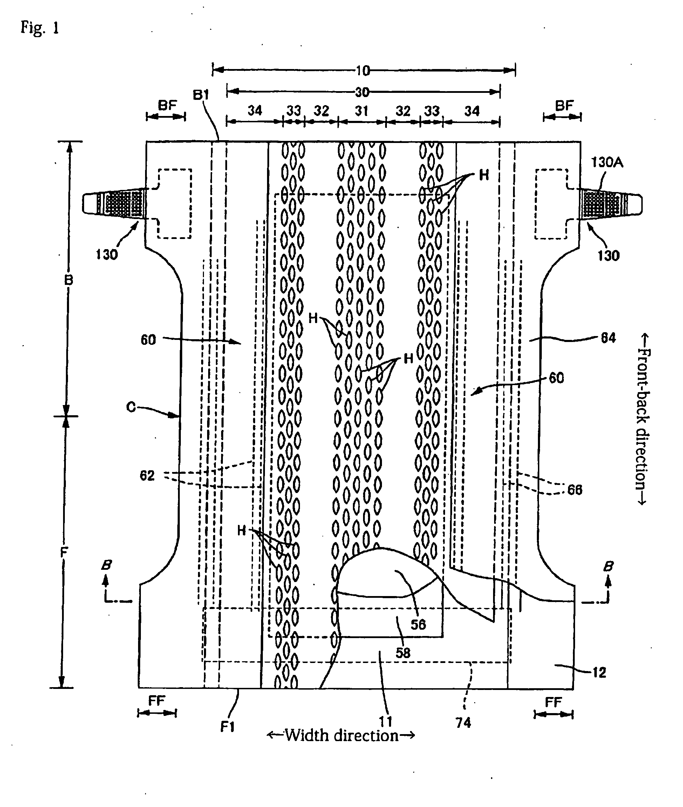

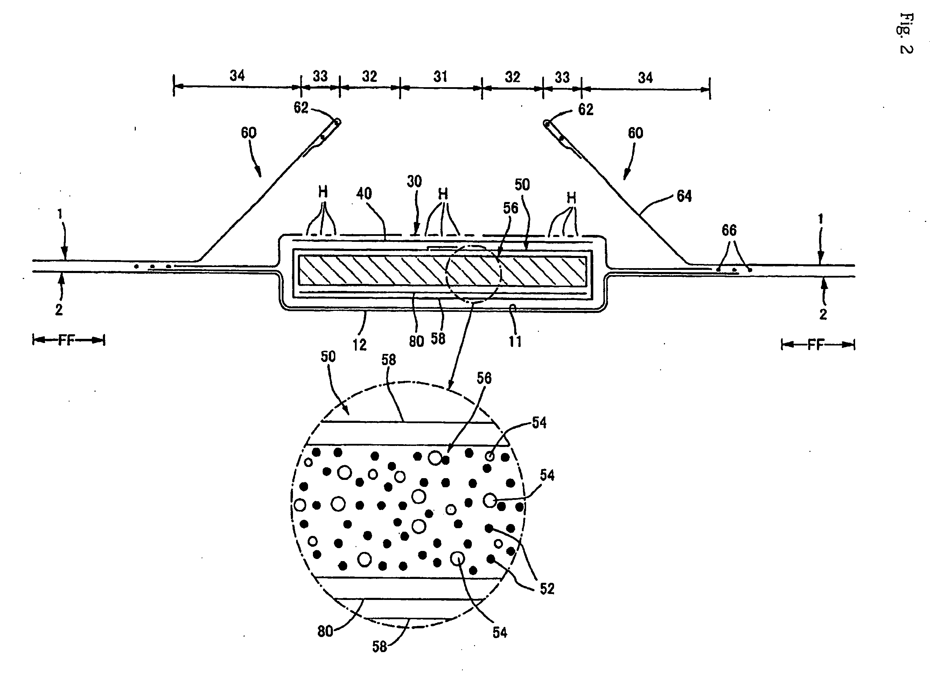

FIGs. 1 and 2 show one example of a tape-type disposable diaper in the present invention. FIG. 2 is a diagram of FIG. 1 along an arrow B-B. The tape-type disposable diaper includes an absorbent part 10 that extends from an upper edge F1 of a ventral-side part F to an upper edge B1 of a back-side part B through a crotch portion C along a center in a width direction, and is designed to absorb and retain excrement; a pair of ventral-side side flap portions FF and FF each extending to an outside of the crotch portion C in the width direction on the both sides of the upper edge F1 of the ventral-side part F; and a pair of back-side side flap portions BF and BF each extending to the outside of the crotch portion C in the width direction on the both sides of the upper edge B1 of the back-side part B. The back-side side flap portions BF and BF are each provided with a fastening piece 130 as a fastening member.
More specifically, entire external surfaces of the absorbent part 10 and the back- and ventral-side side flap portions BF and FF are formed by an outer sheet 12. Particularly in the absorbent part 10, a liquid impervious sheet 11 is fixed to an inner surface of the outer sheet 12 with an adhesive such as a hot-melt adhesive, and an absorbent element 50, an interlayer sheet 40, and a top sheet 30 are layered in this order on an inner surface of the liquid impervious sheet 11. In the illustrated example, the top sheet 30 and the liquid impervious sheet 11 have each the shape of a rectangle extending from the ventral-side upper edge F1 to the back-side upper edge B1, and these sheets are slightly larger in dimensions in the front-back and width directions than the absorbent element 50. The liquid impervious sheet 11 is formed slightly wider than the top sheet 30. Circumferential portions of the top sheet 30 lying off side edges of the absorbent element 50 and circumferential portions of the liquid impervious sheet 11 lying off the side edges of the absorbent element 50 are adhered to each other with a hot-melt adhesive or the like. The top sheet 30 is adhered to the interlayer sheet 40 and/or the absorbent element 50.
Further, the absorbent part 10 has barrier cuffs 60 and 60 projected (erected) from both sides toward the skin of the wearer. Constituting the barrier cuffs 60 and 60, barrier sheets 64 and 64 extend entirely over an outside of the absorbent part 10 in the width direction, including inner surfaces of back- and ventral-side side flap portions BF and FF.
Those components will be described below in sequence.

### (Outer sheet)

The outer sheet 12 is intended to support the absorbent element 50 and is attached to the wearer. The outer sheet 12 has a shape of an hourglass which has a narrow portion at a center portion in the front-back direction, and the wearer's legs are entered into opens formed at the both sides of the narrow portion.
The outer sheet 12 uses preferably a nonwoven fabric, but is not limited to the same. There is no particular limitation on kind of such a nonwoven fabric. For example, raw fibers for the nonwoven fabric may be any of synthetic fibers based on olefin such as polyethylene or polypropylene, polyester, polyamide or the like, recycled fibers such as rayon or cupra, natural fibers such as cotton. In addition, the nonwoven fabric may be produced by any processing method. For example, such a processing method may be a spun lace method, a spun bonding method, a thermal bonding method, an air-through method, a needle punching method, or the like. Preferred is a layered nonwoven fabric such as an SMS nonwoven fabric or an SMMS nonwoven fabric for a combination of favorable texture and sufficient strength. Such a nonwoven fabric may be used singly or a plurality of nonwoven fabrics may be used in a layered manner. In the latter case, the nonwoven fabrics 12 are preferably adhered to each other with a hot-melt adhesive or the like.

### (Top sheet)

The top sheet 30 only needs to have liquid perviousness, and preferably uses a nonwoven fabric from the viewpoints of texture and the like. There is no particular limitation on raw fibers for use in such a nonwoven fabric of the top sheet 30. For example, the raw fibers may be any of synthetic fibers based on olefin such as polyethylene or polypropylene, polyester, polyamide or the like, recycled fibers such as rayon or cupra, natural fibers such as cotton, mixed or composite fibers of two or more of the foregoing fibers. Further, the nonwoven fabric may be produced by any processing method. For example, the processing method may be any publicly known method such as a spun lace method, a spun bonding method, a thermal bonding method, a melt-blown method, a needle punching method, an air-through method, a point bonding method, or the like. Particularly preferred are an air-through nonwoven fabric, a spun-bonded nonwoven fabric, a point-bonded nonwoven fabric, an SMS nonwoven fabric, in which a fineness of raw fibers is 1.0 to 3.0 dtex, and a fiber basis weight is 10 to 30 g/m².
In addition, the top sheet 30 may be a single sheet or a layered sheet obtained by sticking two or more sheets together. Similarly, the top sheet 30 may be a single sheet or two or more sheets in a planar direction.
Permeation holes formed in the top sheet 30 will be described later.

### (Interlayer sheet)

To transfer an excreted matter having passed through the top sheet 30 quickly to the absorbent body and thereby to prevent the back flow phenomenon, an interlayer sheet 40 (also called second sheet) may be interposed between the top sheet 30 and the absorbent element 50. The interlayer sheet 40 allows an excreted matter to move quickly to the absorbent body, thereby to enhance an absorbent capability of the absorbent body. In addition, the interlayer sheet 40 also prevents the absorbed excretion from flowing back from the absorbent body, thereby to keep the surface of the top sheet 30 with a favorable texture. The interlayer sheet 40 may be omitted.
The interlayer sheet 40 may use the same material as that of the top sheet 30, and in particular, uses preferably an air-through nonwoven fabric with high bulkiness and excellent liquid perviousness. The interlayer sheet 40 is preferably joined to the top sheet 30. If the joining is performed using heat embossing or ultrasonic welding, the material for the interlayer sheet 40 preferably has the same level of melting point as that of the top sheet 30. In addition, considering a solid content of stool is allowed to pass through, a preferred fineness of fibers for use in the interlayer sheet 40 is 5.0 to 7.0 dtex, with which, however, a larger amount of liquid is left in the top sheet 30. Meanwhile, if a fineness of fibers for use in the interlayer sheet 40 is 1.0 to 2.0 dtex, a large amount of liquid is less prone to be left in the top sheet 30, but a solid content of stool is less prone to pass through the interlayer sheet 40. Accordingly, a fineness of a nonwoven fabric for use in the interlayer sheet 40 is preferably about 2.0 to 5.0 dtex.
The interlayer sheet 40 is preferably shaped so as to cover all the formation sections of permeation holes H described later, thereby to capture stool having passed through the permeation holes H in a reliable manner. In the illustrated embodiment, the interlayer sheet 40 is centered so as to be shorter than a width of the absorbent element 50, and alternatively the interlayer sheet 40 may be provided so as to cover the entire width of the absorbent element 50. A longitudinal length of the interlayer sheet 40 may be the same as the entire length of the diaper, may be the same as a length of the absorbent element 50, or may be arranged in a short-length area with a liquid-receiving region centered.

FIGs. 11 to 15 propose one preferred embodiment. In this embodiment, the interlayer sheet 40 is interposed between the top sheet 30 and the absorbent element 50 so as to contact an under side surface of the top sheet 30. As illustrated in FIGs. 13 and 14, the interlayer sheet 40 is formed by fixing resilient and elastic members 42 in a stretched state to the base material sheets 41 and 41 with a hot-melt adhesive or the like, and contracting the resilient and elastic members 42, thereby to form wavelike wrinkles on a surface thereof.
In the illustrated embodiment, the two base material sheets 41 and 41 are stuck together with a hot-melt adhesive or the like, and are fixed to each other with the resilient and elastic members 42 interposed therebetween. However, one base material sheet 41 may be used in such a manner that the resilient and elastic members 42 are fixed to either top or under side surface of the base material sheet 41.
The base material sheet 41 may use any of various nonwoven fabrics as the case with the top sheet 30, and in particular, uses preferably an air-through nonwoven fabric with high bulkiness and excellent liquid perviousness. In addition, the base material sheet 41 preferably uses a high-smoothness nonwoven fabric for favorable adhesion to the resilient and elastic members 42. If an air-through nonwoven fabric is used, the base material sheet 41 has a fluffing surface (airy surface) and a smooth surface, and therefore the base material sheet 41 can be adhered on the smooth surface to the resilient and elastic members 42 in a reliable manner. Alternatively, the interlayer sheet may be consolidated under pressure by a press roll or the like such that the fluffing surface is smoothed, thereby allowing reliable adhesion in a similar manner. As stated above, the base material sheet 41 and the resilient and elastic members 42 can be combined to form wavelike wrinkles, thereby to obtain the actually high-bulk interlayer sheet 40 or adjust the interlayer sheet 40 freely in bulkiness. In addition, even if the base material sheet 41 itself is not necessarily excellent in liquid perviousness, it is possible to form wide spaces between the base material sheet 41 and the top sheet 30, which leads to an increase in a possible permeation amount of watery stool and the like. Therefore, the base material sheet 41 can also preferably use a spun-bonded nonwoven fabric and a point-bonded nonwoven fabric which are not high-bulk, an SMS nonwoven fabric and a melt-blown nonwoven fabric which are low in liquid perviousness, and others. These nonwoven fabrics are each manufactured by consolidating with a heat embossing roll, and thus are excellent in smoothness and adhesiveness on both of top and under surfaces. Accordingly, each of the nonwoven fabric are preferred as a material for the base material sheet 41 because the fabric can be used with either the top or under surface as a plane of adhesion to the resilient and elastic members 42 and the fabric also can be formed with wavelike wrinkles in a favorable manner. Further, if the interlayer sheet 40 and the top sheet 30 are adhered to each other by heat embossing or ultrasonic welding, the material for the interlayer sheet 40 has the same level of melting point as that of the top sheet 30. In addition, considering a solid content of stool is allowed to pass through, a preferred fineness of fibers for use in the base material sheet 41 is 5.0 to 7.0 dtex, with which, however, a larger amount of liquid is left in the top sheet 30. Meanwhile, if a fineness of fibers for use in the base material sheet 41 is 1.0 to 2.0 dtex, a large amount of liquid is less prone to be left in the top sheet 30, but a solid content of stool is less prone to pass through the base material sheet 41. Accordingly, a fineness of a nonwoven fabric for use in the base material sheet 41 is preferably about 2.0 to 5.0 dtex.

The resilient and elastic members 42 may be provided in the form of a net or a sheet, and preferably is shaped in the elongated form of threads, strings, bands or the like so as to suppress a decrease in liquid perviousness.
If the interlayer sheet 40 is formed by sticking together the two base material sheets 41 and 41 of nonwoven fabrics and interposing elongated resilient and elastic members between the sheets 41 and 41, the nonwoven fabrics each preferably has a fineness of 2.0 to 5.0 dtex (in particular 3.0 to 4.0 dtex), a basis weight of fibers of 10 to 30 g/m² (in particular 15 to 20 g/m²), and a thickness of 0.1 to 4.0 mm (in particular 0.5 to 2.0 mm). In addition, the resilient and elastic members 42 preferably use synthetic rubber threads with a fineness of 150 to 1,240 dtex (in particular 450 to 600 dtex). Preferably, the resilient and elastic members 42 are fixed in parallel in a stretched state of 100 to 300% (in particular 150 to 200%) at intervals Z of 3 to 15 mm (in particular 5 to 10 mm) and are contracted to thereby form wavelike wrinkles on the surface of the interlayer sheet 40 in a direction crossing with the resilient and elastic members 42. Accordingly, the interlayer sheet 40 has long and regular wrinkles formed to allow spaces between the interlayer sheet 40 and the top sheet 30 to be made continuous in a longer distance.

Preferably, the wrinkles have each a depth Y of about 1 to 5 mm, and a wrinkle peak-to-peak distance X of about 1 to 10 mm. The wrinkles can be formed by adjusting as appropriate a material and a thickness of the base material sheet 41 and an extension ratio of the resilient and elastic members 42.
A direction of formation of the wrinkles (orthogonal to the direction of stretch of the resilient and elastic members) can be decided as appropriate. The wrinkles may be formed along the front-back direction of the diaper, and preferably are formed along the width direction of the diaper because watery stool and the like are less prone to be spread in the front-back direction, thereby preventing effectively the urination parts and others of the wearer from being soiled with the stool.

In the interlayer sheet 40, all the wrinkles on the surface do not need to be positioned on the under side of the permeation holes H in the top sheet 30. It is only necessary that at least some of the wrinkles are positioned on the under side of the permeation holes H in the top sheet 30. A wrinkled section of the interlayer sheet 40 can be arranged as appropriate, and preferably is arranged so as to contain a section opposed to the anus of the wearer. Specifically, for the diapers for babies and infants, the interlayer sheet 40 has preferably a wrinkled section so as to contain a front-back area ranging from a position of 50 mm frontward to a position of 160 mm backward with respect to the center of the diaper in the front-back direction, and contain a widthwise area ranging from a position of 50 mm leftward to a position of 50 mm rightward with respect to the center of the diaper in the width direction.

The interlayer sheet 40 may be wrinkled partly or entirely. The wrinkled section of the interlayer sheet 40 can be modified by changing appropriately an area in which the elastic members 42 are fixed in a stretched state, or by fixing the resilient and elastic members 42 in a stretched state across the full length of the base material sheet 41 and then processing by an arbitrary method an unwrinkled part out of the section with the resilient and elastic members 42 so as not to be made resilient or elastic. In particular, if the interlayer sheet 40 is to have an unwrinkled section and have wrinkled sections on front and back sides of the unwrinkled section, the elastic members 42 may be cut finely by a method described in JP 2002-035029 A or JP 2002-178428 A so that the interlayer sheet 40 is partly unwrinkled. To facilitate manufacture of the interlayer sheet 40 and fixation of the interlayer sheet 40, the interlayer sheet 40 is preferably unwrinkled on both end portions of the elastic members 42 in a direction of stretching (the front-back end portions in the illustrated embodiment).

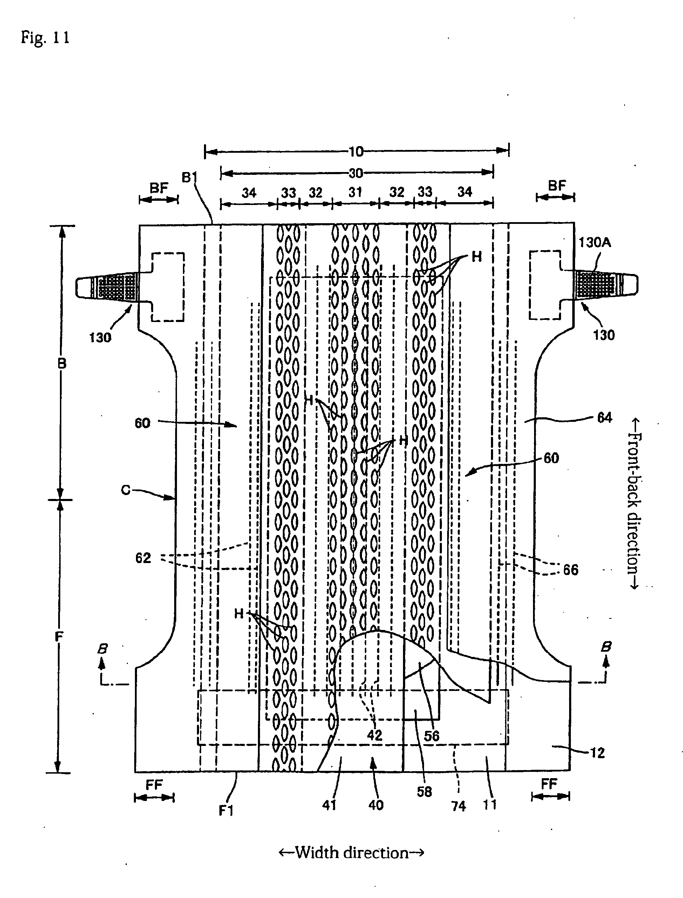

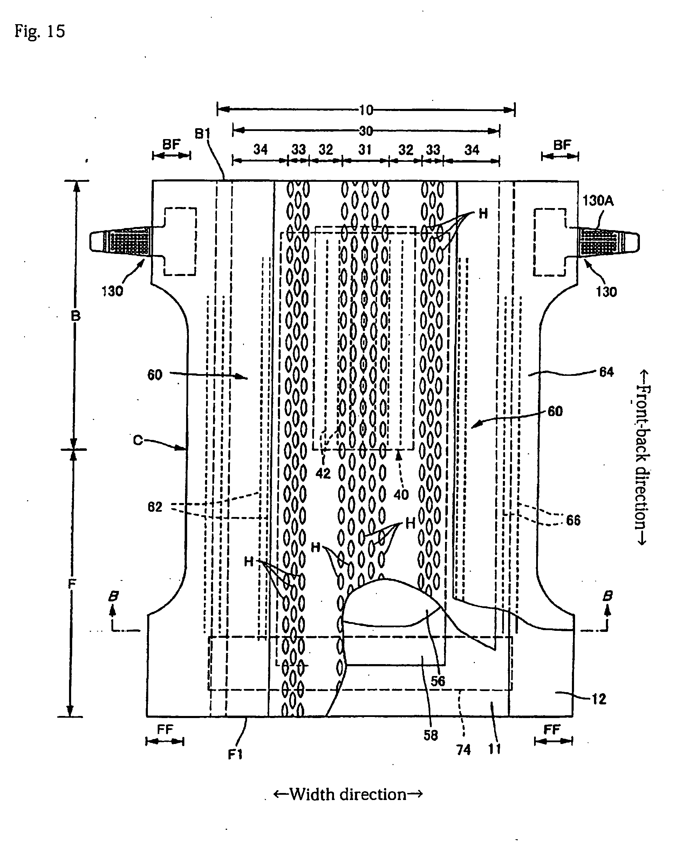

In the embodiment shown in FIG. 11, the interlayer sheet 40 is centered so as to be shorter than the width of the absorbent element 50, and the interlayer sheet 40 does not extend to the under sides of the permeation hole groups of the left- and right-side regions 33. Alternatively, the interlayer sheet 40 may be provided across the full width of the absorbent element 50 so as to cover the under sides of the permeation hole groups. In addition, the length of the interlayer sheet 40 in the front-back direction of the diaper is preferably identical to the entire length of the diaper as shown in FIG. 11 for ease of manufacture, or may be identical to the length of the absorbent element 50. Alternatively, the interlayer sheet 40 may have a shorter-length area in which a region for receiving stool is centered as shown in FIG. 15, for example, an area ranging from the central portion of the diaper in the front-back direction to the back end portion of the absorbent body.
With interposition of the interlayer sheet 40, linearly continuous spaces 43 are formed between concave parts of the wrinkles on the interlayer sheet 40 and the top sheet 30, as described above. Accordingly, watery stool and the like having passed through the permeation holes H in the top sheet 30 are received and spread in the spaces 43, whereby the watery stool and the like are less prone to spread on the surface of the top sheet 30 and soiling of the skin of the wearer is decreased. Further, since the interlayer sheet 40 are wrinkled by contraction forces of the elastic members 42, even if the interlayer sheet 40 is temporarily stretched out by external pressure, the interlayer sheet 40 is forcedly returned to the original state upon removal of the external pressure, thereby to ensure the above-mentioned spaces 43 between the interlayer sheet 40 and the top sheet 30.
The foregoing embodiment of the wrinkled interlayer sheet 40 has the permeation holes H in the top sheet 30, but the interlayer sheet 40 of the present invention is also applicable to an embodiment with no permeation holes in the top sheet 30 (not shown). In addition, although the permeation holes H in the foregoing embodiment are characteristic, the interlayer sheet 40 of the present invention has no particular limitations in layout, shape, or structure of the permeation holes H in the top sheet 30, and may also employ any publicly known arrangement.

### (Barrier cuffs)

To block urine and soft stool moving laterally over the top sheet 30 and thereby prevent lateral leakage, the barrier cuffs 60 and 60 are preferably erected on the both sides of the diaper on a surface touching the skin of the wearer.
The barrier cuffs 60 each include a barrier sheet 64 made continuous in a virtually lateral direction, and an elongated resilient and elastic member 62 fixed in a stretched state to the barrier sheet 64 in the front-back direction. The barrier sheet 64 may use a water-repellent nonwoven fabric. The resilient and elastic member 62 may use rubber threads or the like. The resilient and elastic member 62 may be plurally provided to each of the cuffs as shown in FIGs. 1 and 2 for example, or may be singly provided to each of the cuffs.
The barrier sheet 64 has adhesion start points in an inner surface thereof above the side portions of the top sheet 30. The barrier sheets 64 are adhered, with a hot-melt adhesive or the like, from the adhesion start points outward in the width direction to the side portions of the liquid impervious sheet 11 and the side portions of the outer sheet 12 located on the outside of the liquid impervious sheet 11. In these adhered sections, resilient and elastic members 66 such as rubber threads are provided in the front-back direction in the vicinities of the adhesion start points between the barrier sheet 64 and the outer sheet 12 which are opposed to each other.
Around the leg parts, the barrier cuffs 60 are fixed from the adhesion start points inward in the width direction to the top sheet 30 on the both ends of the diaper in the front-back direction. The barrier cuffs 60 have each an unfixed free section between the fixed sections. The free section is erected by the action of contraction forces of the rubber threads 62. In wearing of a diaper, when the diaper is applied to the body of the wearer in a boat-like form, the barrier cuffs 60 are erected by the action of contraction forces of the rubber threads 62 to thereby fit the diaper closely to the wearer around the legs. This prevents lateral leakage from the legs of the wearer.

### (Absorbent element)

The absorbent element 50 is a component to absorb and retain liquids such as urine and loose stool. The absorbent element 50 has the absorbent body 56 and an envelope sheet 58 for enveloping at least under and side surfaces of the absorbent body 56. The envelope sheet 58 may be omitted. Further, the illustrated embodiment has a holding sheet 80 provided between the absorbent body 56 and an under side portion (lower portion) of the envelope sheet 58, but the holding sheet 80 may be omitted. The absorbent element 50 may be adhered on an under surface thereof to the inner surface of the liquid impervious sheet 11, with an adhesive such as a hot-melt adhesive.

### (Absorbent body)

The absorbent body 56 can be formed by an assembly of fibers 52 and 52. Such a fiber assembly may use accumulated short fibers such as fluff pulp, synthetic fibers or the like, or may use a filament assembly that can be obtained by opening as necessary a tow (fiber bundle) of synthetic fibers made of cellulose acetate or the like. A basis weight of fibers may be about 100 to 300 g/m² for accumulated fluff pulp or short fibers, for example, and may be about 30 to 120 g/m² for a filament assembly, for example. A fineness of synthetic fibers is 1 to 16 dtex, preferably 1 to 10 dtex, more preferably 1 to 5 dtex, for example. If a filament assembly is used, filaments may be non-crimped fibers but preferably are crimped fibers. A degree of crimping of the crimped fibers may be about 5 to 75 crimps per inch, preferably about 10 to 50 crimps per inch, and more preferably about 15 to 50 crimps per inch. In many cases, uniformly crimped fibers are used.

### (High-absorbent polymer particles)

Preferably, the absorbent body 56 contains high-absorbent polymer particles 54, 54 ..., as shown in FIG. 2. In particular, at least in an area receiving a liquid, high-absorbent polymer particles (SAP particles) are desirably scattered in a virtually overall thickness direction with respect to the assembly of fibers 52, 52 ....
If there are no or few if any, SAP particles in upper, lower and central portions of the absorbent body 56, it is not recognized that "the SAP particles are scattered in the overall thickness direction". Therefore, the state "scattered in the overall thickness direction" refers to a mode in which the particles are scattered "evenly" in the overall thickness direction with respect to the assembly of fibers, or a mode in which the particles are "unevenly distributed" in the upper, lower and/or central portions but still are scattered in the upper, lower and/or central portions. In addition, the foregoing state does not exclude a mode in which some of the SAP particles does not enter into the assembly of fibers 52, 52 ... and remain on a surface of the same, or a mode in which some of the SAP particles pass through the assembly of fibers 52, 52 ... and exist on the envelope sheet 58 or the holding sheet 80.
The high-absorbent polymer particles 54 may be not only "particles" but also "powders". A particle diameter of the high-absorbent polymer particles 54 may be the same as that of particles used in this kind of absorbent articles, and is 1,000 µm or less, desirably in particular 150 to 400 µm. There are no particular limits on a material for the high-absorbent polymer particles 54, and a preferred material is 40 g/g or more in capacity of water absorption. The high-absorbent polymer particles 54 may be based on starch, cellulose or synthetic polymer, and may use starch-acrylic acid (salt) graft copolymer, saponified product of starch-acrylonitrile copolymer, cross-linked sodium carboxymethyl cellulose, acrylic acid (salt) polymer, or the like. A shape of the high-absorbent polymer particles 54 is preferably a commonly used particulate shape, and may also be any other shape.
The high-absorbent polymer particles 54 preferably used are a water absorption speed of 40 seconds or less. If the water absorption speed exceeds 40 seconds, a backflow phenomenon becomes prone to occur, where a liquid supplied to the absorbent body 56 flows back out of the absorbent body 56.
A basis weight of the high-absorbent polymer particles 54 may be decided as appropriate in accordance with an absorption capacity required for the absorbent body 56, and may be 50 to 350 g/m², although it is not always defined so. If the basis weight is lower than 50g/m², it is difficult to maintain the absorption capacity. If the basis weight exceeds 350 g/m², the high-absorbent polymer particles 54 become saturated in effectiveness and an excessive amount thereof gives an unpleasant grainy feel.

### (Envelope sheet)

The envelope sheet 58 may use any of materials such as tissue paper, particularly crepe paper, nonwoven fabrics, polyethylene-laminated nonwoven fabrics, foraminous sheets, and the like. The sheet desirably does not let high-absorbent polymer particles pass through. If a nonwoven fabric is used for the envelope sheet 58 instead of crepe paper, a hydrophilic SMMS (spun bonded/melt-blown/melt-blown/spun-bonded) nonwoven fabric is preferred in particular. A material for such a fabric may be polypropylene, polyethylene/polypropylene, or the like. A basis weight of the fabric is desirably 5 to 40 g/m², particularly 10 to 30 g/m².
The envelope sheet 58 may be configured as to envelop an overall layer containing the assembly of continuous fibers 52, 52 ... and the high-absorbent polymer particles 54, 54 ... as shown in FIG. 2, or may envelop only under and side surfaces of the layer. Further, although not shown, the envelope sheet 58 may be configured as to cover the upper and side surfaces of the absorbent body 56 with crepe paper or nonwoven fabric, and cover the under surface of the same with a liquid impervious sheet of polyethylene or the like, or as to cover the upper surface of the absorbent core 56 with crape paper of nonwoven fabric and side and under surfaces of the absorbent core 56 with liquid impervious sheet of polyethylene or the like (the foregoing materials are constitutional elements of the envelope sheet). If necessary, the envelope sheet 58 may be configured in such a manner that the layer containing the assembly of continuous fibers 52, 52 ... and the high-absorbent polymer particles 54, 54... is sandwiched between two upper and lower sheets, or in such a manner that one sheet is disposed only on the lower surface of the layer. However, these configurations are not desired because they make it difficult to prevent movement of the high-absorbent polymer particles.

### (Holding sheet)

If a filament assembly is used as the fibers 52, the holding sheet 80 is preferably provided. In providing the holding sheet 80, the high-absorbent polymer particles 54 may be interposed by dispersing or the like between the holding sheet 80 and the absorbent body 56. The high-absorbent polymer particles 54 may pass through the filament assembly during a process of supply to the filament assembly, a process subsequent to the foregoing process, or a process of distribution to consumers. The high-absorbent polymer particles having passed through the filament assembly may bring an unpleasant grainy feel with asperities thereof to a user who touches the product by hand.
The holding sheet 80 performs the functions of increasing elasticity which would not be sufficiently provided by the envelope sheet 58 alone made of tissue paper (crepe paper) or the like, and reducing or preventing an uncomfortable feel given to a user who touches the product by hand.
There is no particular limitation on a material for the holding sheet 80, and such a material only needs to be capable of holding the high-absorbent polymers 54. Specifically, the material may be any of nonwoven fabrics, crimped pulp, low-absorbent cotton fibers (e.g. fat cotton fibers, defatted cotton fibers, rayon fibers processed with a water repellent agent or a hydrophobizing agent), polyethylene fibers, polyester fibers, acrylic fibers, polypropylene fibers, silk, cotton, linen, nylon, polyurethane, acetate fibers, and the like, for example.
The holding sheet 80 has preferably a thickness larger than a diameter of a high-absorbent polymer particle. In addition, a basis weight of the holding sheet 80 is preferably 10 to 60 g/m², in particular 20 to 40 g/m².
In particular, the holding sheet 80 is formed by a nonwoven fabric with compression energy of 0.01 to 10.00 gfcm/cm², preferably 0.01 to 1.00 gfcm/cm², and compression resilience of 10 to 100%, preferably 70 to 100%, on the basis of test results from KES Test.
In addition, since the slipped high-absorbent polymers 54 are held by the holding sheet 80 and thus do not move over the envelope sheet 58. This makes the holding sheet 80 less prone to exercise uneven absorption performance. Particularly, in order to prevent movement of the high-absorbent polymer particles 54 over the holding sheet 80, the holding sheet 80 may be coated in advance with a sticky hot-melt adhesive or the like. Alternatively, in order to prevent movement of the high-absorbent polymer particles 54 over the holding sheet 80, an upper surface of the holding sheet 80 (facing the used surface of the absorbent body 56) may be made rough. For this purpose, the nonwoven fabric may be manufactured in such a manner that a surface thereof is roughed or fluffed by making non-netted, marbling, needle-punching, or brushing.
The holding sheet 80 may be provided only underneath the absorbent body 56 as shown in FIG. 2, or may pass by the absorbent body 56, roll and extend to the upper surface of the absorbent body 56, although not shown. In addition, a plurality of holding sheets 80 may be stacked.
Although, in the above example, the holding sheet 80 is disposed between the absorbent body 56 and the envelope sheet 58 on the lower side, the holding sheet may be placed under the envelope sheet 58 instead (this arrangement is not shown). The important point is that providing the holding sheet 80 under the absorbent body 56 reduces or eliminates an unpleasant grainy feel which would be given to a user who touches the product from the under surface thereof.

### (Fastening pieces)

The fastening pieces 130 each have a base portion of a fastening base material 130C made of plastic, polyethylene-laminated nonwoven fabric, paper or the like, connected to the diaper, and have a hook member 130A of the mechanical fastener on a leading end of the base portion, as an engagement portion with respect to the ventral-side part. The hook member 130A is connected with an adhesive to the fastening base material 130C in such a manner that the two cannot be separated. The hook member 130A has a large number of engagement projections on an outer surface thereof. A shape of the engagement projections may be any of (A) a a V letter", (B) a J letter, (C) a mushroom, (D) a T letter, (E) a double-J letter (in which two J's are joined back to back) and the like. Instead of the hook member 130A, an adhesive material layer may be arranged as an engagement portion of the fastening piece 130.
In wearing of the diaper, the back-side flap portion BF is placed over the ventral-side flap portion FF on an outside, and the fastening piece is engaged with the ventral-side part F in an appropriate position on an external surface. A position and size of an engagement part of the fastening piece 130 can be decided arbitrarily. In general, preferably, the engagement position falls within a rectangular area 20 to 80 mm high and 150 to 300 mm wide, and a separation distance in the height direction between an upper end edge of the engagement area and the ventral-side upper edge F1 is 0 to 60 mm, in particular 20 to 50 mm, and the engagement area is centered in the width direction of the diaper.
Preferably, a target tape 74 is disposed on the ventral-side part F at the engagement position of the fastening piece 130 for facilitation of engagement. The target tape 74 may have a large number of thread loops on a surface thereof for engagement with the engagement projections of the hook element 130A. If an adhesive layer is used as an engagement portion, the target tape 74 may be a smooth film with an excellent adherence property.
In addition, if the ventral-side part F has the engagement portion made of a nonwoven fabric with respect to the fastening piece 130, for example, if the outer sheet 12 in the illustrated embodiment is formed by a nonwoven fabric and the engagement portion of the fastening piece 130 is formed by the hook element 130A, the hook element 130A may be engaged with the nonwoven fabric of the outer sheet 12 without the use of the target tape 74.

### (Permeation holes in the top sheet)

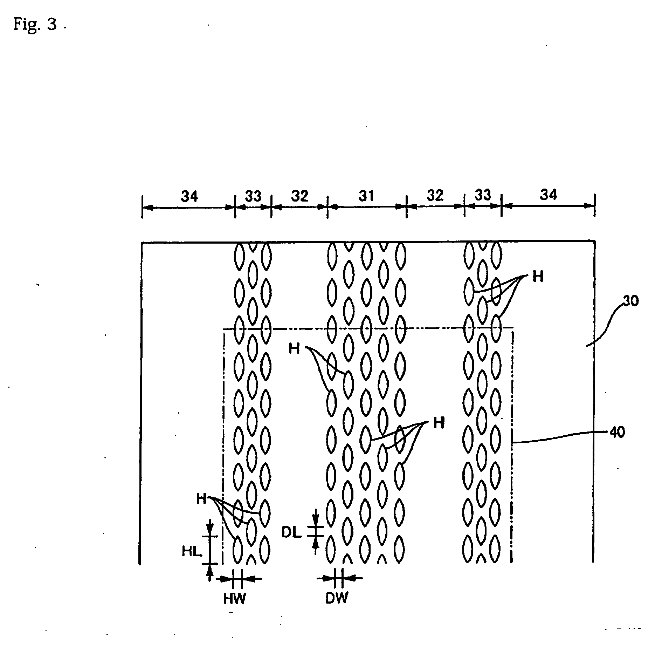

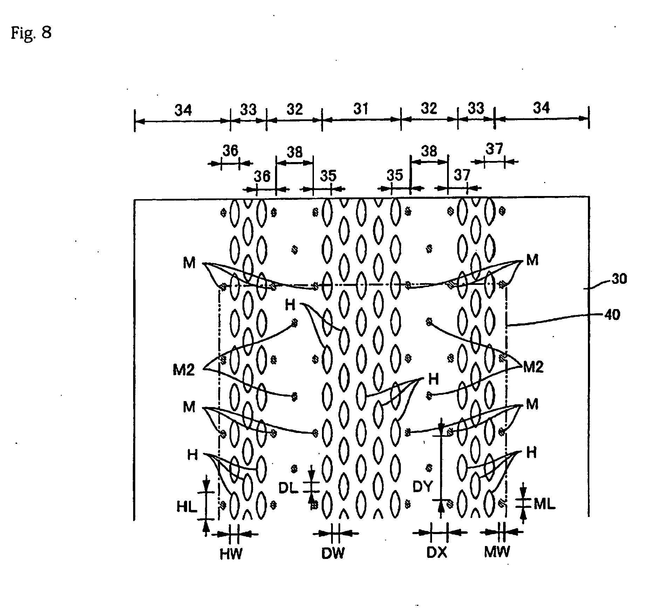

With reference to correspondence with an intergluteal cleft and the tops of the left and right gluteal regions, the top sheet 30 includes the following regions: a widthwise central region 31 that extends from a front end F1 to a back end B1 through a portion corresponding to the intergluteal cleft; left- and right-side intermediate regions 32 and 32 that are positioned on left and right sides of the widthwise central region 31 and extend from the front end F1 to the back end B1 through sections corresponding to the top of the left gluteal region and the top of the right gluteal region, respectively; and left- and right-side regions 33 and 33 that extend from the front end F1 to the back end B1 along the left side of the left-side intermediate region 32 and the right side of the right-side intermediate region 32, respectively. Further, in the illustrated embodiment, the top sheet 30 includes side edge regions 34 and 34 that contain portions for joining and fixing the top sheet 30 to the liquid impervious sheet 11 on the left side of the left-side region and on the right side of the right-side region. The left- and right-side regions 33 are positioned on the absorbent element 50.
Specifically, in the diapers for babies and infants, an appropriate width of the widthwise central region 31 is about 20 to 40 mm, appropriate widths of the left- and right-side intermediate regions 32 are each about 15 to 25 mm, and appropriate widths of the left- and right-side regions 33 are each about 10 to 20 mm.
In addition, characteristically as shown in FIG. 3 (also applicable to the embodiments of FIGs. 8 to 10), the top sheet 30 has a large number of permeation holes H for letting a solid content in watery stool and the like pass through toward an under side thereof, at predetermined intervals in the widthwise central region 31 and the left-and right-side regions 33. In addition, the top sheet 30 has no permeation holes H in the entire left- and right-side intermediate regions 32 and 32. Therefore, coverage areas of the widthwise central region 31 and left- and right-side regions 33 are determined in such a manner that the widthwise arrangement of perforated and unperforated areas of the permeation holes H applies at least to sections corresponding to the intergluteal cleft, tops of left and right gluteal regions, and to sections on the outsides of the foregoing sections. Only part of each of the regions 31 to 33 in the front-back direction may have the foregoing perforated and unperforated areas arranged in the width direction, as far as the perforated and unperforated areas contain the sections corresponding to the intergluteal cleft, the tops of left and right gluteal regions, and the sections on the outsides of the foregoing sections. For example, only the back-side part B may employ the foregoing pattern.
In addition, the widths of the left- and right-side regions 33 and 33 are preferably smaller than the width of the widthwise central region 31, because when the wearer lies down on his/her side, a decreased amount of excretion flows back to the gluteal region of the wearer through the permeation holes H in the left- or right-side region 33 contacting the left or right gluteal region of the wearer.

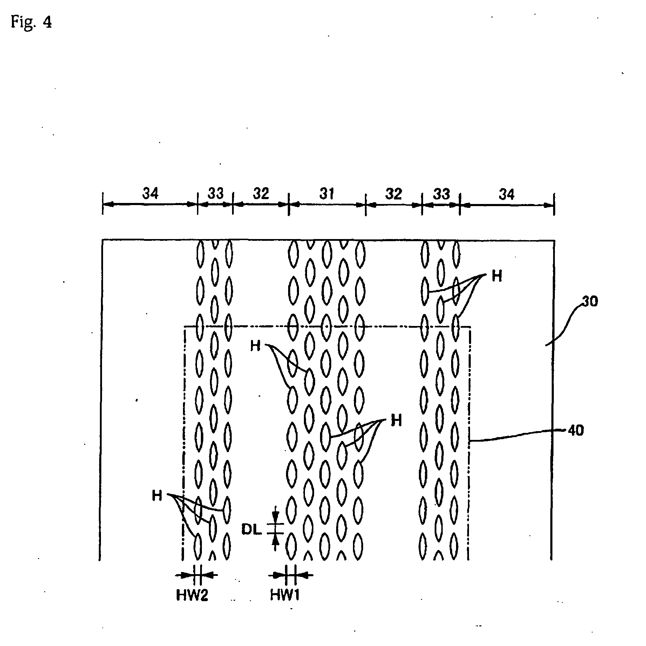

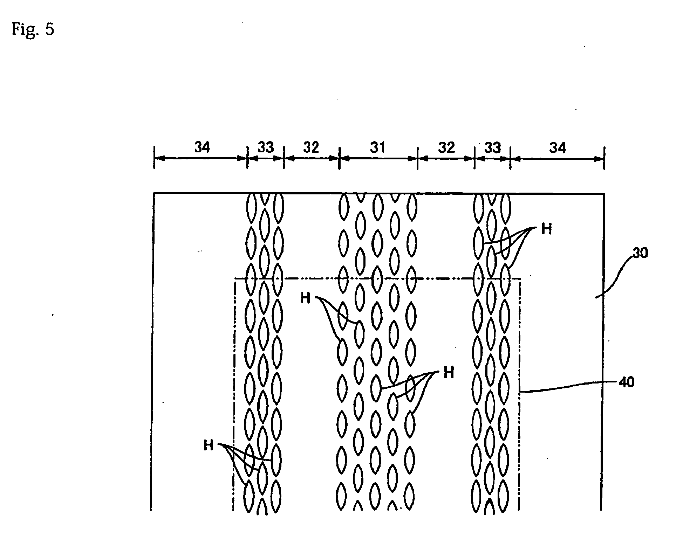

The permeation holes H may have any of appropriate opening shapes such as a round, an ellipse, an oval, a bamboo leaf, and a polygon. In particular, the permeation holes H preferably has an elongated shape (an ellipse, an oval, or a bamboo leaf) in which a front-back length HL is 4 to 15 mm, in particular 6 to 8 mm, and a widthwise length HW is shorter than the front-back length HL.
An opening area of the permeation hole H can be decided as appropriate, and is preferably 0.8 to 180 mm², more preferably 5 to 80 mm², most preferably 10 to 30 mm². An excessively small opening area would decrease the diaper in a capability of capturing a solid content, and an excessively large opening area would make stool prone to flow back through the permeation holes H.
In addition, the number of the permeation holes H can be decided as appropriate. As shown in the diagram, if permeation hole lines in which the permeation holes H with the above-mentioned opening area are aligned in the front-back direction at predetermined intervals, are provided at predetermined intervals in the width direction, and if the permeation holes H have the opening area stated above, the number and layout of the permeation holes H are preferably decided for each of the regions 31 to 33 in such a manner that a widthwise interval DW between lines of the permeation holes H is 1 to 5 mm and a front-back interval DL between the permeation holes H in a line is 1 to 10 mm. If the intervals between the permeation holes H are excessively wide, a solid content of stool is prone to move without being captured. If the intervals are excessively narrow, the permeation holes H are increased in number and cause stool to flow back easily through the permeation holes H. In addition, the permeation holes H may be arranged in a zigzag pattern (as illustrated), a grid pattern (line-column pattern), or any other regular or irregular pattern.
The permeation holes H formed in the widthwise central region 31 and the permeation holes H formed in the left- and right-side regions 33 and 33 may be identical or different in shape, dimensions, and arrangement interval. In one preferred embodiment, as shown in FIG. 4, a lengthwise opening length HW1 of the permeation hole H in the widthwise central region 31 is 4 to 10 mm; a widthwise opening length HW2 of the permeation hole H in the left- and right-side regions 33 and 33 is 2 to 7 mm, which is shorter than that in the widthwise central region 31. As stated above, when the widthwise opening length HW1 of the permeation hole H in the left- and right-side regions 33 and 33 is shorter than that in the widthwise central region, a total area of the permeation holes contacting the right or left gluteal region of the wearer lying down on his/her side becomes small. This provides an advantage that a decreased amount of excretion flows back through the permeation holes H and attaches to the right or left gluteal region of the wearer.
In another preferred embodiment, as shown in FIG. 5, an opening area of the permeation hole H in the widthwise central region 31 is 5 to 40 mm², an opening area of the permeation hole H in the left- and right-side regions 33 and 33 is 10 to 50 mm², which is larger than that in the widthwise central region 31. Accordingly, it is possible to effectively capture stool with a high solid content having reached the left- and right-side regions 33 and 33.

### (Fixation of the top sheet)

The top sheet 30 is desirably fixed at the unperforated section to the member on the under side thereof. In particular, to provide a wider space for capturing stool, it is preferred that the top sheet 30 is not adhered to the member on the under side thereof at least at a widthwise central portion in the perforated section. The top sheet 30 can be fixed by a welding method such as ultrasonic welding, heat sealing, or heat embossing, or by an adhering method using an adhesive such as a hot-melt adhesive. However, since it is not preferred that the adhesive is exposed from the permeation holes H, the top sheet 30 is preferably welded to the member on the under side thereof in an area of ± 10 mm or less, in particular ±5 mm or less, from the both side edges of the permeation hole groups in the width direction.

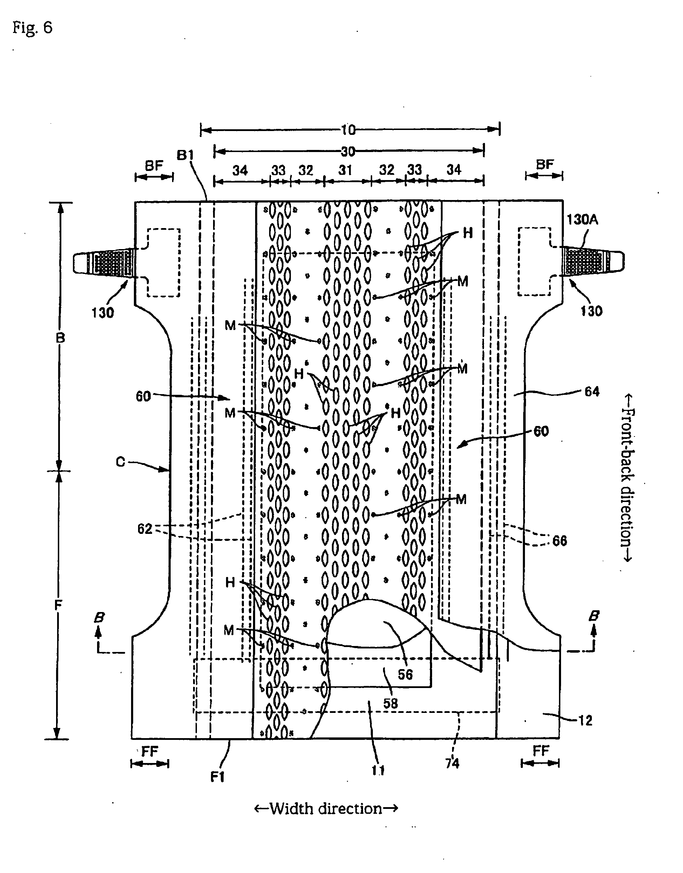

In one preferred embodiment, as shown in FIGs. 6 to 10, a large number of welding points M are disposed at predetermined intervals in first to third sections 35 to 37 of ± 10 mm or less in the width direction from the both side edge of the permeation hole groups in the width direction (in the illustrated arrangement, the widthwise central region 31, and the left- and right-side regions 33 and 33), thereby to fix the top sheet 30 to the members (in the illustrated arrangement, the interlayer sheet 40, the absorbent element 50, and the liquid impervious sheet 11. This applies to the subsequent descriptions.) on the under side thereof in the areas without the use of an adhesive such as a hot-melt adhesive for fixation of the top sheet 30 to the member on the under side thereof. That is, the top sheet 30 is fixed in the first to third sections only by material welding. Further, the top sheet 30 is not fixed to the members on the under side thereof at sections with the permeation holes (in the illustrated embodiment, the widthwise central region 31, and the left- and right-side regions 33 and 33) excluded from a scope of the first to third sections 35 to 37.

Therefore, the sections of the top sheet 30 with the permeation hole groups can be fixed in width by the welding points M and M on the both sides thereof, thereby to suppress breakage of the permeation holes H. In addition, the top sheet 30 has the sections between the welding points M and M in which clearances are created between the sections with the permeation holes H and the members (in the illustrated embodiment, mainly the interlayer sheet 40) on the under side thereof, whereby watery stool and the like can be easily captured into the top sheet 30 through the permeation holes H. Here, the first to third sections 35 to 37 are defined as being ± 10 mm or less in the width direction from the corresponding reference side edges, making allowance for displacements of the top sheet 30 inevitably caused at formation of the welded points M. The sections 35 to 37 are each more preferably in an area of ± 5 mm or less, further more preferably an area of ± 2.5 mm or less. In particular, the first to third sections 35 to 37 are preferably configured in such a manner that the welded points M and the permeation holes H are close to each other in the width direction but not overlap each other (in other words, the welded points M are positioned in the sections 35 to 37 on the outsides of the reference side edges in the width direction).

Sections other than the sections with the permeation hole groups and the welded points M, for example, a widthwise intermediate section 38 in the left-side intermediate region 32, and a widthwise intermediate section 38 in the right-side intermediate region 32 can be fixed by provision of intermediate region welded points M2 or by use of an adhesive such as a hot-melt adhesive instead of the intermediate region welded points M2, for enhancement of fixation strength of the top sheet 30.
The welded points M and the intermediate region welded points M2 can be formed by an appropriate method such as ultrasonic welding, heat sealing, or heat embossing, as far as those welded points allow the materials to be joined by welding. At manufacturing, the top sheet 30 and the members on the under side thereof can be layered and adhered to each other by a joining process such as ultrasonic welding.
The welding point M can have any of appropriate planar shapes such as a round, an ellipse, an oval, a bamboo leaf, and a polygon. In general, the welding point M preferably has an elongated shape (an ellipse, an oval, or a bamboo leaf) in which a front-back length ML is 1 to 10 mm, in particular 1 to 3 mm, and a widthwise length MW is shorter than the front-back length ML.
An area of the welded point M can be decided as appropriate, and is preferably 0.7 to 40 mm², in particular preferably 1 to 15 mm². If the welded area is excessively small, it is difficult to provide the top sheet 30 with sufficient fixation strength. In contrast, if the welded area is excessively large, the welded points M and the permeation holes H are likely to overlap in the first to third sections 35 to 37.

In addition, the number of the welded points M can be decided as appropriate. As shown in the diagram, if welded point lines in which the welded points M are aligned at predetermined intervals in the front-back direction, are provided at predetermined intervals in the width direction, and if the welded area is as stated above, a front-back interval DY between the welded points M in a welded point line is preferably 5 to 30 mm, more preferably 5 to 15 mm, in the regions 31 to 33. If pluralities of welded points M and M2 are provided in each of the regions 31 to 33, a widthwise interval DX between the lines is 2 to 15 mm, more preferably 4 to 10 mm. If the intervals between the welded points M are excessively wide, the permeation holes are likely to be broken. If the intervals between the same are excessively narrow, the sheet becomes hard in texture. In addition, the welded points M may be arranged in line in the sections 35 to 37 in the front-back direction at equal intervals as in the illustrated example, or when the plurality of the welded points are provided with intervals in the width direction, the welded points M may be arranged in a zigzag pattern (as illustrated), a grid pattern (line-column pattern), or any other regular or irregular pattern.

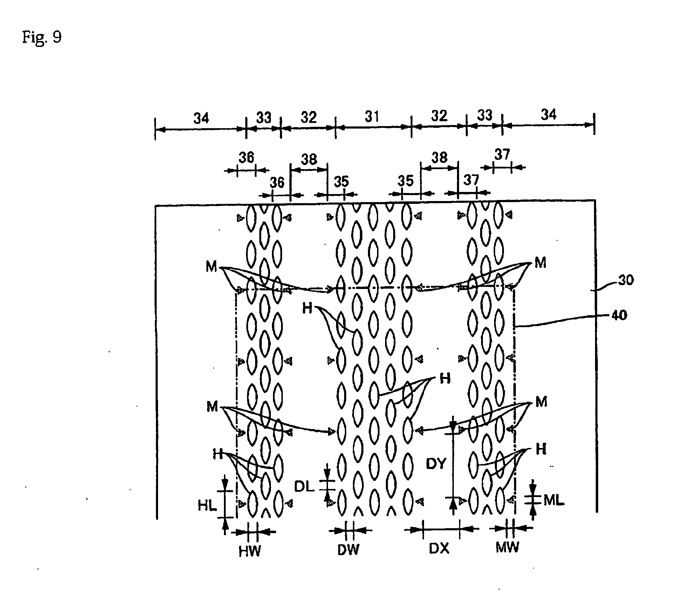

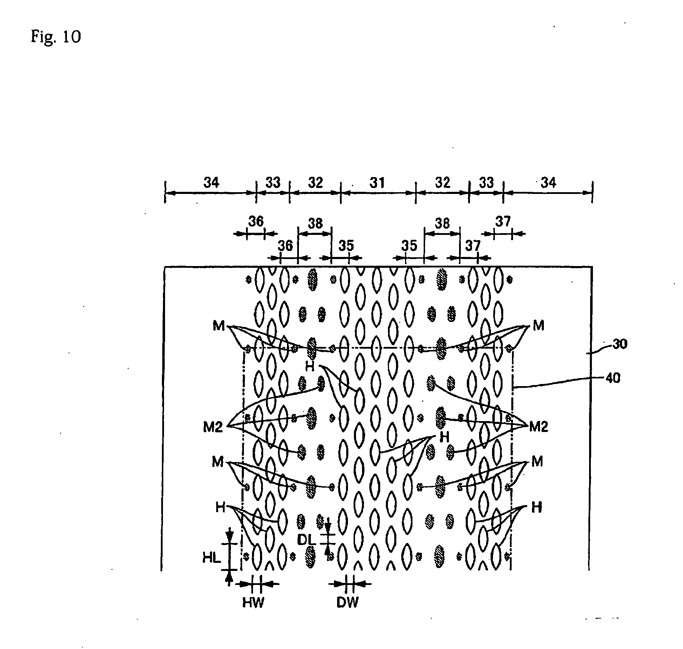

The welded points M and the intermediate region welded points M2 may be different in shape, dimensions, arrangement interval, depending on the widthwise positions thereof. In one preferred embodiment as shown in FIG. 9, out of the welded points M arranged in the first to third sections 35 to 37, the welded points M located on the left side of the reference permeation hole groups in the sections 35 to 37 in the width direction, are longer in the front-back direction with distance leftward in the width direction from the reference permeation hole groups, and the welded points M located on the right side of the reference permeation hole groups in the width direction, are longer in the front-back direction with distance rightward in the width direction from the reference permeation hole groups.
In this embodiment, occupied areas of the welded points M are larger with distance in the width direction from the side edges of the reference permeation hole groups, and therefore the welded points M and the permeation holes H are less prone to overlap or the welded points M and the permeation holes H overlap only by limited areas even if the top sheet 30 is displaced in the width direction at a welding process. In addition, since the occupied areas of the welded points M are larger with distance from the side edges of the reference permeation hole groups, the welded points M increases the top sheet 30 in fixation strength with distance from the side edges of the permeation hole groups, which compensates for decrease in fixation strength of the top sheet 30 to some extent at the unfixed sections.
In addition, the intermediate region welded points M2 may be basically arranged in the same manner as the welded points M in the first to third sections, in shape, area, arrangement interval, arrangement pattern, and the like, or may be arranged in a different manner. In one preferred embodiment as shown in FIG. 10, the areas of the intermediate welded points M2 may be larger than those of the welded points M in the first to third sections 35 to 37. Further, as shown in FIG. 10, the intermediate region welded points M2 in the left-side intermediate region 32, the welded points M in the first to third sections 35 to 37 on the both sides of the left-side intermediate region 32, the intermediate region welded points M2 in the right-side intermediate region 32, and the welded points M in the first to third sections 35 to 37 on the both sides of the right-side intermediate region 32, are larger in area with increasing proximity to the centers of the intermediate regions 32 in the width direction.

By making the areas of the intermediate region welded points M2 larger than the welded portions M in the first to third sections 35 to 37 as stated above, it is possible to further increase the top sheet 30 in fixation strength and liquid absorption rate. In addition, since the left- and right-side intermediate regions 32 and 32 under pressure from the tops of the left and right gluteal regions are welded by larger areas, backflow of a liquid is less prone to take place at those intermediate regions.

### (Methods for perforating the top sheet)

The top sheet 30 with the permeation holes H can be manufactured by a method in which the sheet is punched in a predetermined shape at the permeation hole formation sections; a method in which the sheet is fused and perforated by a heat roller with a plurality of projections (JP 2002-512909 A); a method in which the sheet is three-dimensionally tapered and perforated in a suction stretch thermal process by an open conveyor under negative pressure (JP 2812340 B); or a method in which the sheet is formed with slits in a predetermined direction (front-back or width direction or the like) at the permeation hole formation positions, the sheet is stretched in a direction orthogonal to the slits, and then the sheet is fixed, while the slits are thus extended, to the member on the under side thereof (the interlayer sheet 40 or the absorbent element 50) (JP H11-253490 A). The foregoing methods have their respective merits, but also have their respective demerits. Specifically, the punching method generates material waste. The fusing and perforating method makes the texture of the sheet harder at circumferences of the holes. The three-dimensional tapering method makes the holes prone to be broken by application of a pressing force in the thickness direction. The slitting and stretching method makes the holes prone to be loose and closed when the diaper is bent. Accordingly, the top sheet 30 is most preferably manufactured by the method in which the sheet is formed with slits in the front-back direction at the permeation hole formation positions; the sheet is stretched in the width direction; and then the sheet is fixed, while the slits are thus extended, to the member on the under side thereof, in combination with other techniques described below. This allows the top sheet 30 to offer excellent performance while eliminating the foregoing demerits.

The sheet, manufactured by the method in which the sheet is provided with slits in the front-back direction at the permeation hole formation positions; the sheet is stretched in the width direction; and then the slits are extended to thereby form holes, is not hardened by melt-solidification of the opening and the circumferential thereof in thermal processing, and is soft in texture and excellent in liquid perviousness owing to widened inter-fiber gaps. However, since wide spaces are provided for retaining stool on the under side of the top sheet 30 as stated above, if the top sheet 30 and the member on the under side thereof are not fixed across the almost full width thereof, the extended holes in the top sheet 30 become loose and prone to be closed when the diaper is bent. In contrast, if the top sheet 30 is fixed to the member over the almost entire surface thereof, it is not possible to form spaces for stool retention on the under side of the top sheet 30. Therefore, the top sheet 30 is preferably adhered to the member at specific intervals in the width direction. By non-perforating the sheet at left and right intermediate regions, instead of perforating the sheet along the almost full width as stated above, it is possible to prevent attachment of back-flow excretion to the gluteal region of the wearer. In addition, by using the right and left intermediate regions (and the both side portions of the top sheet 30) to adhere the top sheet 30 to the member on the under side thereof, it is possible to fix the top sheet 30 firmly while keeping the permeation holes H in an open state. In this case, it is preferred to adhere the top sheet 30 at regions in the vicinities of the both side edges of the permeation hole groups in the width direction, thereby maintaining the opening of the permeation holes H in a stable manner. In particular, the top sheet 30 is preferably adhered by welding at the foregoing welded points M.

### Industrial Applicability

The present invention can be utilized for tape-type disposable diapers and other types of disposable diapers such as underpants-type disposable diapers.

### Brief Description of the Drawings

FIG. 1 is a plane view of an opened tape-type disposable diaper;
FIG. 2 is a cross-section view of FIG. 1 along line B-B;
FIG. 3 is an enlarged plane view of main components of FIG. 1;
FIG. 4 is an enlarged plane view of main components showing permeation holes of another example;
FIG. 5 is an enlarged plane view of main components showing permeation holes of still another example;
FIG. 6 is a plane view of an opened tape-type disposable diaper;

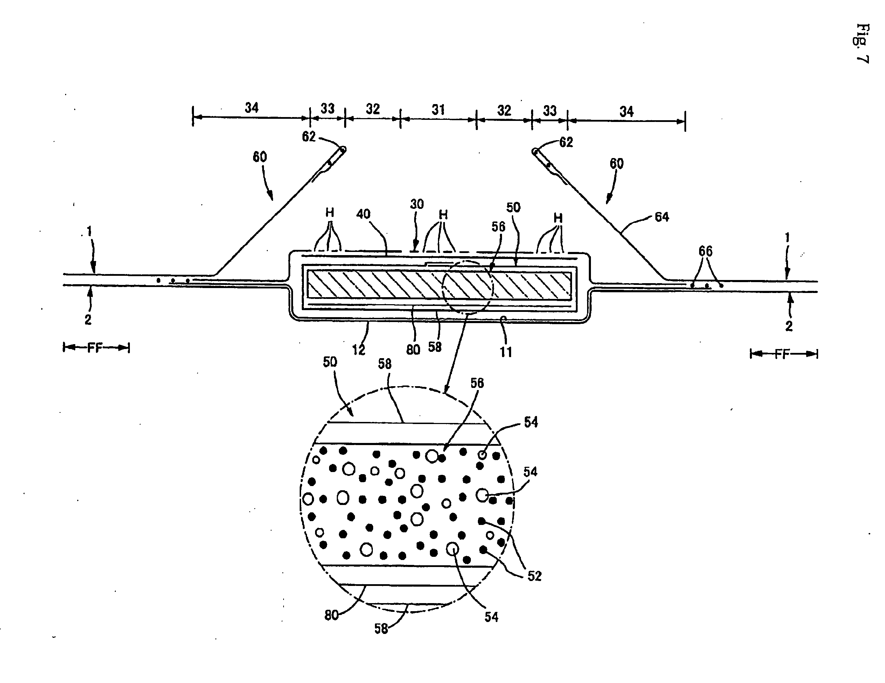

FIG. 7 is a cross-section view of FIG. 6 along line B-B;
FIG. 8 is an enlarged plane view of main components of FIG. 6;
FIG. 9 is an enlarged plane view of main components showing another example of welding points;
FIG. 10 is an enlarged plane view of main components showing still another example of welding points;
FIG. 11 is a plane view of an opened tape-type disposable diaper;

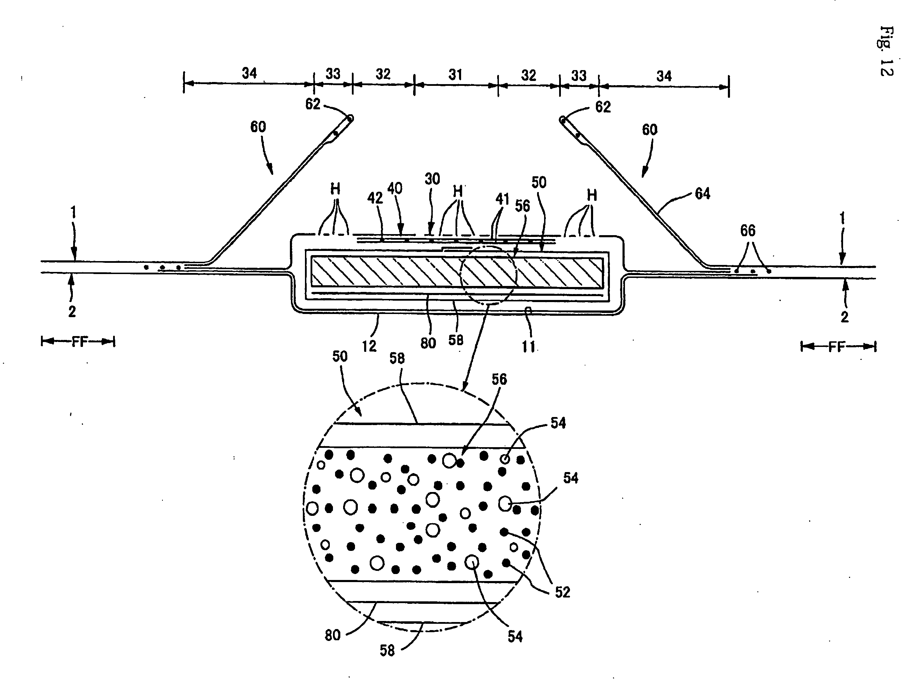

FIG. 12 is a cross-section view of FIG. 11 along line B-B;

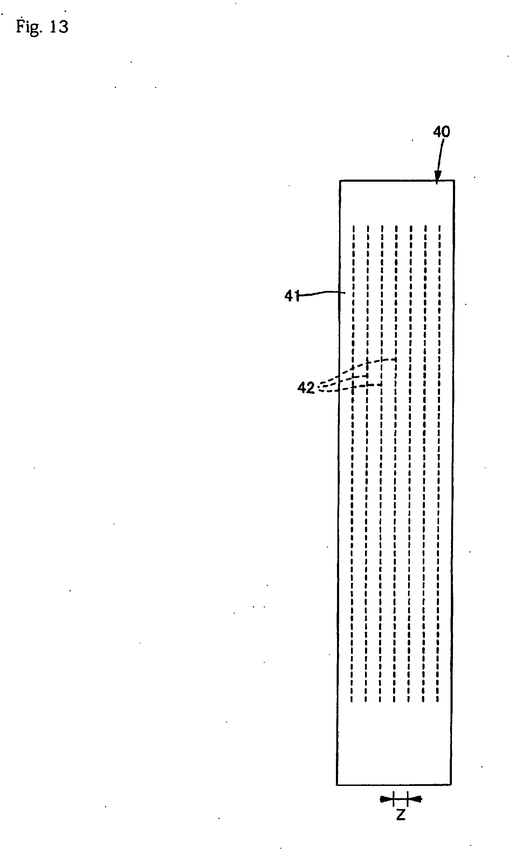

FIG. 13 is a plane view of an interlayer sheet;

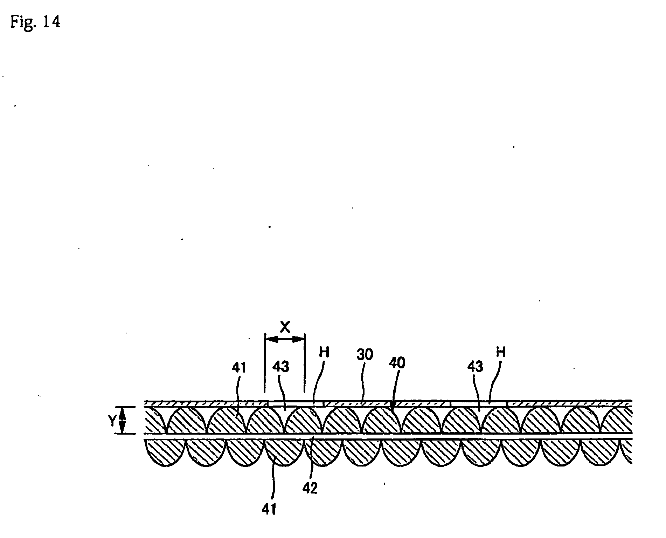

FIG. 14 is an enlarged cross-section view of a top sheet and the interlayer sheet; and
FIG. 15 is a plane view of another opened tape-type disposable diaper.

### Description of the reference numerals

10...Absorbent part, 11... Liquid impervious sheet, 12... Outer sheet, 30... Top sheet, 31... Widthwise central region, 32... Left-side intermediate region, right-side intermediate region, 33... Left-side region, right-side region, 34... Side-edge region, 40... Interlayer sheet, 41... Base material sheet, 42... Elastic member, 43... Space, 50... Absorbent element, 52... Fiber, 54... High-absorbent polymer particle, 56...Absorbent body, 58... Envelope sheet, 60... Barrier cuff, 64... Barrier sheet, 80... Holding sheet, H... Permeation hole, M, M2... Welding point

## Claims

1. A disposable diaper, comprising an absorbent part (10) which has a liquid pervious top sheet (30) constituting a surface facing the body of a wearer, a liquid impervious sheet (11) located on an external side, an absorbent element (50) interposed between the liquid pervious top sheet and the liquid impervious sheet, and which extends from an upper edge (F1) of a ventral side (F) through a crotch portion (C) to an upper edge (B1) of the back side (B) along a center in the width direction, wherein
the top sheet (30) has a widthwise central region (31) extending from front to back ends through a portion corresponding to an intergluteal cleft; left- and right-side intermediate regions (32) that are positioned on left and right sides of the widthwise central region and extend from front to back ends through portions corresponding to tops of left and right gluteal regions and, respectively; and left - and right -side regions (33) that extend from front to back ends along a left side of the left-side intermediate region and a right side of the right-side intermediate region, respectively,
a large number of permeation holes (H) are formed at predetermined intervals in the widthwise central region at least at an entire widthwise section in a front-back direction corresponding to the intergluteal cleft; in the left-side region at least at an entire widthwise section in the front-back direction corresponding to a section located on a left side of the top of the left gluteal region in the width direction; and in the right-side region at least at an entire widthwise section in the front-back direction corresponding to a section located on a right side of the top of the right gluteal region in the width direction, and no permeation hole is formed in the left-side intermediate region at least at a section corresponding to the top of the left gluteal region and in the right-side intermediate region at least in a section corresponding to the top of the right gluteal region.

2. The disposable diaper according to Claim 1, wherein
a width of the right- and left-side regions is smaller than a width of the widthwise central region.

3. The disposable diaper according to Claim 1, wherein
the top sheet is formed by a nonwoven fabric with a fineness of raw fibers of 1.0 to 3.0 dtex and a fiber basis weight of 10 to 30 g/m²,
the permeation holes are arranged in such a pattern that permeation hole lines with the permeation holes aligned at predetermined intervals in the front-back direction, are provided at predetermined intervals in the width direction,
an opening area of the permeation hole is 0.8 to 180 mm², a widthwise interval between the permeation hole lines is 1 to 5 mm, and a front-back interval between the permeation holes in the permeation hole lines is 1 to 10 mm, in each of the widthwise central region and the right- and left-side regions.

4. The disposable diaper according to Claim 3, wherein
the permeation hole has an elongated opening shape in which a front-back length (HL) is 4 to 15 mm and a widthwise length (HW) is smaller than the front-back length.

5. The disposable diaper according to Claim 1, wherein
the permeation holes in the right- and left-side regions have each a widthwise opening length of 2 to 7 mm, and the permeation holes in the widthwise central region have each a widthwise opening length of 4 to 10 mm, and the widthwise length of the permeation holes in the right- and left-side regions is smaller than the widthwise length of the permeation holes in the widthwise central region.

6. The disposable diaper according to Claim 1, wherein
the permeation holes in the widthwise central region have each an opening area of 5 to 40 mm², the permeation holes in the right- and left-side regions have each an opening area of 10 to 50 mm², and the opening area of each of the permeation holes in the right- and left-side regions is larger than the opening area of each of the permeation holes in the widthwise central region.

7. The disposable diaper according to Claim 1, wherein
the top sheet has left- and right-side side edge regions extending from front to back ends along a left side of the left-side region and a right side of the right-side region, respectively,
no permeation hole is formed in the left- and right-side side edge regions, and
a large number of welded points (M) are provided at predetermined intervals to fix the top sheet to a member on an under side thereof, in a first section of ± 10 mm or less in the width direction from both side edges of the permeation hole group in the width direction in the widthwise central region; in a second section of ± 10 mm or less in the width direction from both side edges of the permeation hole group in the left-side region; and in a third section of ± 10 mm or less in the width direction from both side edges of the permeation hole group in the right-side region, the top sheet is fixed to the member on the under side thereof without using an adhesive, and the widthwise central region and the left- and right-side regions are not fixed to the member on the under side excluded from a scope of the first to third sections.

8. The disposable diaper according to Claim 7, wherein
the permeation holes are formed by providing front-back slits in the top sheet at permeation hole formation positions and stretching the top sheet in the width direction to thereby extend the slits, and the top sheet is adhered, while the slits are thus extended, to a member on the under side of the top sheet, at regions in vicinities of the both side edges of the permeation hole groups in the width direction.

9. The disposable diaper according to Claim 7, wherein
the top sheet is formed by a nonwoven fabric with a fineness of raw fibers of 1.0 to 3.0 dtex and a fiber basis weight of 10 to 30 g/m²,
the permeation holes are arranged in such a pattern that permeation hole lines with the permeation holes aligned at predetermined intervals in the front-back direction, are provided at predetermined intervals in the width direction,
the welded points are arranged in such a pattern that welded point lines with the welded points aligned at predetermined intervals in the front-back direction, are provided at predetermined intervals in the width direction,
an opening area of the permeation hole is 0.8 to 180 mm², a widthwise interval between the permeation hole lines is 1 to 5 mm, and a front-back interval between the permeation holes in the permeation hole lines is 1 to 10 mm, in each of the widthwise central region and the right- and left-side regions, and
an area of the welded point is 0.7 to 40 mm², a widthwise interval between the welded point lines is 2 to 15 mm, and a front-back interval between the welded points in the welded point lines is 5 to 30 mm.

10. The disposable diaper according to Claim 7, wherein
in the first section, occupied areas of the welded points on a left side of the permeation hole group in the width direction in the widthwise central region are larger with distant from the permeation hole group in the widthwise central region toward the left in the width direction; in the first section, occupied areas of the welded points on a right side of the permeation hole group in the width direction in the widthwise central region are larger with distant from the permeation hole group in the widthwise central region toward the right in the width direction,
in the second section, occupied areas of the welded points on a left side of the permeation hole group in the width direction in the left-side region are larger with distant from the permeation hole group in the left-side region toward the left in the width direction; and in the second section, occupied areas of the welded points on a right side of the permeation hole group in the width direction in the left-side region are larger with distant from the permeation hole group in the left-side region toward the right in the width direction, and
in the third section, occupied areas of the welded points on a left side of the permeation hole group in the width direction in the right-side region are larger with distant from the permeation hole group in the right-side region toward the left in the width direction; and in the third section, occupied areas of the welded points on a right side of the permeation hole group in the width direction in the right-side region are larger with distant from the permeation hole group in the right-side region toward the right in the width direction.

11. The disposable diaper according to Claim 7, wherein
out of the welded points in the first section, the welded points on the left side of the permeation hole group in the width direction in the widthwise central region have each a shape that is larger in a front-back length with distant from the permeation hole group in the widthwise central region toward the left in the width direction; and out of the welded points in the first section, the welded points on the right side of the permeation hole group in the width direction in the widthwise central region have each a shape that is larger in a front-back length with distant from the permeation hole group in the widthwise central region toward the right in the width direction,
out of the welded points in the second section, the welded points on the left side of the permeation hole group in the width direction in the left-side region have each a shape that is larger in a front-back length with distant from the permeation hole group in the left-side region toward the left in the width direction; and out of the welded points in the second section, the welded points on the right side of the permeation hole group in the width direction in the left-side region have each a shape that is larger in a front-back length with distant from the permeation hole group in the left-side region toward the right in the width direction, and
out of the welded points in the third section, the welded points on the left side of the permeation hole group in the width direction in the right-side region have each a shape that is larger in a front-back length with distant from the permeation hole group in the right-side region toward the left in the width direction; and out of the welded points in the third section, the welded points on the right side of the permeation hole group in the width direction in the right-side region have each a shape that is larger in a front-back length with distant from the permeation hole group in the right-side region toward the right in the width direction.

12. The disposable diaper according to Claim 7, wherein
a large number of intermediate region welded points are provided at predetermined intervals to fix the top sheet to a member on the under side thereof, in the left- and right-side intermediate regions excluded from a scope of the first to third sections, and
areas of the intermediate region welded points are larger than the areas of the welded points in the first to third sections.

13. The disposable diaper according to Claim 1, further comprising:
an interlayer sheet (40) between the top sheet and the absorbent element so as to contact the under side of the top sheet, wherein
the interlayer sheet has wavelike wrinkles formed by fixing elastic members (42) in a stretched state to a base material sheet (41), and then contracting the base material sheet by contraction of the elastic members.

14. The disposable diaper according to Claim 13, wherein
the interlayer sheet is formed by sticking together two nonwoven fabrics with a fineness of 2.0 to 5.0 dtex, a fiber basis weight of 10 to 30 g/m², and a thickness of 0.1 to 4.0 mm; fixing a plurality of elongated resilient and elastic members with a fineness of 150 to 1,240 dtex in parallel in a 100 to 300% stretched state and at intervals of 3 to 15 mm between the foregoing nonwoven fabrics; and contracting the base material sheet by contraction of the elastic members, thereby to form wavelike wrinkles on the base material sheet surface in a direction crossing with the elastic members.

15. The disposable diaper according to Claim 14, wherein
the wrinkles are formed in a diaper width direction.

16. A disposable diaper according to claim 1, wherein,
an interlayer sheet (40) is interposed between the top sheet and the absorbent element so as to contact the under side of the top sheet,
the interlayer sheet is formed by fixing elastic members (42) in a stretched state to a base material sheet (41) and contracting the base material sheet by contraction of the elastic members, thereby to form wavelike wrinkles on the base material sheet surface, and the wrinkles are at least partly located on the under side of the permeation holes in the top sheet.

17. The disposable diaper according to Claim 16, wherein
the interlayer sheet is formed by sticking together two nonwoven fabrics with a fineness of 2.0 to 5.0 dtex, a fiber basis weight of 10 to 30 g/m², and a thickness of 0.1 to 4.0 mm; fixing a plurality of elongated resilient and elastic members with a fineness of 150 to 1,240 dtex in parallel in a 100 to 300% stretched state and at intervals of 3 to 15 mm between the foregoing nonwoven fabrics; and contracting the base material sheet by contraction of the elastic members, thereby to form wavelike wrinkles on the base material sheet surface in a direction crossing with the elastic members.

18. The disposable diaper according to Claim 17, wherein
the wrinkles are formed in a diaper width direction.

## Patentansprüche

1. Einwegwindel mit einem absorbierenden Teil (10), das eine flüssigkeitsdurchlässige Deckschicht (30), die eine Oberfläche bildet, die dem Körper eines Trägers zugewandt ist, eine flüssigkeitsundurchlässigen Schicht (11), die an einer Außenseite angeordnet ist, ein absorbierendes Element (50) hat, das zwischen der flüssigkeitsdurchlässigen Deckschicht und der flüssigkeitsundurchlässigen Schicht eingefügt ist, und das sich von einer oberen Kante (F1) einer ventralen Seite (F) durch einen Schrittabschnitt (C) zu einer oberen Kante (B1) der Rückseite (B) entlang eines Zentrums in Richtung der Breite erstreckt, wobei,
die Deckschicht (30) einen breitenmäßigen Zentralbereich (31), der sich vom vorderen bis zum rückseitigen Ende durch einen Abschnitt erstreckt, der einer Pospalte entspricht; links- und rechtsseitige Zwischenbereiche (32), die auf den linken und rechten Seiten des in Richtung der Breite gelegenen Zentralbereichs positioniert sind und sich vom vorderen zum rückseitigen Ende durch Abschnitte erstrecken, die den Oberseiten von linken beziehungsweise rechten Gesäßregionen entsprechen; und links- und rechtsseitige Bereiche (33) hat, die sich vom vorderen zum rückseitigen Ende entlang einer linken Seite des linksseitigen Zwischenbereichs beziehungsweise einer rechten Seite des rechtsseitigen Zwischenbereichs erstrecken,
eine große Anzahl von Durchdringungslöchern (H) in vorbestimmten Intervallen in der breitenmäßigen Zentralbereich wenigstens an einem vollständigen breitenmäßigen Abschnitt in einer Richtung von vorne nach hinten gebildet sind, der der Pofalte entspricht; im linksseitigen Bereich wenigstens an einem vollständigen breitenmäßigen Abschnitt in der Richtung von vorne nach hinten gebildet sind, der einem Abschnitt entspricht, der auf einer linken Seite des Oberteils der linken Gesäßregion in Breitenrichtung angeordnet ist; und im rechtsseitigen Bereich zumindest an einem vollständigen breitenmäßigen Abschnitt in der Richtung von vorne nach hinten entsprechend einem Abschnitt gebildet sind, der auf einer rechten Seite der Oberseite der rechten Gesäßregion in Breitenrichtung angeordnet ist, und kein Durchdringungsloch in dem linksseitigen Zwischenbereich wenigstens an einem Abschnitt entsprechend der Oberseite der linken Gesäßregion und in dem rechtsseitigen Zwischenbereich zumindest in einem Abschnitt entsprechend der Oberseite der rechten Gesäßregion gebildet ist.

2. Einwegwindel nach Anspruch 1, wobei eine Breite der rechts- und linksseitigen Bereiche kleiner ist als eine Breite des breitenmäßigen Zentralbereichs.

3. Einwegwindel nach Anspruch 1, wobei die Deckschicht gebildet ist aus einem nicht verwebten Stoff mit einer Feinheit der Rohfasern von 1,0 bis 3,0 dtex und einem Faserbasisgewicht von 10 bis 30 g/m²,
die Durchdringungslöcher in solch einem Muster angeordnet sind, dass Durchdringungslochlinien mit den Durchdringungslöchern, die in vorbestimmten Intervallen in der Richtung von vorne nach hinten ausgerichtet sind, in vorbestimmten Intervallen in der Breitenrichtung vorgesehen sind,
ein Öffnungsbereich des Durchdringungslochs der 0,8 bis 180 mm² beträgt, ein breitenmäßiges Intervall zwischen den Durchdringungslochlinien 1 bis 5 mm beträgt und ein Intervall von vorne nach hinten zwischen den Durchdringungslöchern in den Durchdringungslochlinien 1 bis 10 mm beträgt in jedem der Bereiche umfassend den breitenmäßigen Zentralbereiche und die rechts- und linksseitigen Bereiche.

4. Einwegwindel nach Anspruch 3, wobei das Durchdringungsloch eine längliche Öffnungsform hat, bei der eine Länge (HL) von vorne nach hinten 4 bis 15 mm beträgt und eine breitenmäßige Länge (HW) kleiner als die Länge von vorne nach hinten ist.

5. Einwegwindel nach Anspruch 1, wobei die Durchdringungslöcher in den rechts- und linksseitigen Bereichen jeweils eine breitenmäßige Öffnungslänge von 2 bis 7 mm haben und die Durchdringungslöcher im breitenmäßigen Zentralbereich jeweils eine breitenmäßige Öffnungslänge von 4 bis 10 mm haben und die breitenmäßige Länge der Durchdringungslöcher in den rechts- und linksseitigen Bereichen kleiner ist als die breitenmäßige Länge der Durchdringungslöcher im breitenmäßigen Zentralbereich.

6. Einwegwindel nach Anspruch 1, wobei die Durchdringungslöcher im breitenmäßigen Zentralbereich jeweils einen Öffnungsbereich von 5 bis 40 mm² haben, die Durchdringungslöcher in den rechts- und linksseitigen Bereichen jeweils einen Öffnungsbereich von 10 bis 50 mm² haben und der Öffnungsbereich von jedem der Durchdringungslöscher in den rechts- und linksseitigen Bereichen größer ist als der Öffnungsbereich jedes der Durchdringungslöcher im breitenmäßigen Zentralbereich.

7. Einwegwindel nach Anspruch 1, wobei
die Deckschicht links- und rechtsseitige Seitenkantenbereich hat, die sich vom vorderen zum rückseitigen Ende entlang einer linken Seite des linksseitigen Bereichs bzw. einer rechten Seite des rechtsseitigen Bereichs erstrecken,
kein Durchdringungsloch in den links- und rechtsseitigen Seitenkantenbereichen gebildet ist, und
eine große Anzahl von Schweißpunkten (M) an vorbestimmten Intervallen vorgesehen sind, um die Deckschicht an einem Element an einer Unterseite davon zu fixieren, in einem ersten Abschnitt von +/- 10 mm oder weniger in der Breitenrichtung von beiden Seitenkanten der Durchdringungslochgruppe in der Breitenrichtung im breitenmäßigen Zentralbereich; in einem zweiten Abschnitt von +/- 10 mm oder weniger in der Breitenrichtung von beiden Seitenkanten der Durchdringungslochgruppe im linksseitigen Bereich; und in einem dritten Abschnitt von +/- 10 mm oder weniger in der Breitenrichtung von beiden Seitenkanten der Durchdringungslochgruppe im rechtsseitigen Bereich, wobei die Deckschicht am Element an der Unterseite ohne Verwendung eines Klebers fixiert ist und der breitenmäßige Zentralbereich und die links- und rechtsseitigen Bereiche nicht an dem Element an der Unterseite befestigt sind, die von einem Bereich der ersten bis dritten Abschnitte ausgeschlossen sind.

8. Einwegwindel nach Anspruch 7, wobei die Durchdringungslöcher durch Vorsehen von Schlitzen von vorne nach hinten in der Deckschicht an Durchdringungslochbildungspositionen und Dehnen der Deckschicht in der Breitenrichtung gebildet sind, um dadurch die Schlitze zu erweitern, und die Deckschicht, während die Schlitze so erweitert sind, an einem Element an der Unterseite der Deckschicht festgehalten ist in Bereichen in der Nachbarschaft der beiden Seitenkanten der Durchdringungslochgruppen in der Breitenrichtung.

9. Einwegwindel nach Anspruch 7, wobei
die Deckschicht durch einen nicht verwebten Stoff mit einer Feinheit an Rohfasern von 1,0 bis 3,0 dtex und einem Fasergewicht von 10 bis 30 g/m² geformt ist,
die Durchdringungslöcher in so einem Muster angeordnet sind, dass Durchdringungslochlinien mit den Durchdringungslöchern, die in vorbestimmten Intervallen in einer Richtung von vorne nach hinten ausgerichtet sind, in vorbestimmten Intervallen in der Breitenrichtung vorgesehen sind,
die Schweißpunkte in so einem Muster angeordnet sind, dass Schweißpunktlinien mit den Schweißpunkten, die in vorbestimmten Intervallen in der Richtung von vorne nach hinten ausgerichtet sind, in vorbestimmten Intervallen in der Breitenrichtung vorgesehen sind,
ein Öffnungsbereich des Durchgangslochs 0,8 bis 180 mm² beträgt, ein breitenmäßiges Intervall zwischen den Durchgangslochlinien 1 bis 5 mm beträgt und ein Intervall von vorne nach hinten zwischen den Durchgangslöchern in den Durchgangslochlinien 1 bis 10 mm beträgt, in jedem Bereich des breitenmäßigen Zentralbereichs und der rechts- und linksseitigen Bereiche, und
ein Bereich des Schweißpunkts 0,7 bis 40 mm² beträgt, ein breitenmäßiges Intervall zwischen den Schweißpunktlinien 2 bis 15 mm beträgt und ein Intervall von vorne nach hinten zwischen den Schweißpunkten in den Schweißpunktlinien 5 bis 30 mm beträgt.

10. Einwegwindel nach Anspruch 7, wobei
im ersten Abschnitt besetzte Bereiche der Schweißpunkte auf einer linken Seite der Durchdringungslochgruppe in Breitenrichtung in dem breitenmäßigen Zentralbereich größer sind mit Abstand von der Durchdringungslochgruppe im breitenmäßigen Zentralbereich nach links in der Breitenrichtung; im ersten Abschnitt besetzte Bereich der Schweißpunkte auf einer rechten Seite der Durchdringungslochgruppe in Breitenrichtung im breitenmäßigen Zentralbereich größer sind mit Abstand von der Durchdringungslochgruppe im breitenmäßigen Zentralbereich nach rechts in der Breitenrichtung,
im zweiten Abschnitt besetzte Bereiche der Schweißpunkte auf einer linken Seite der Durchdringungslochgruppe in der Breitenrichtung im linksseitigen Bereich größer sind mit Abstand von der Durchdringungslochgruppe im linksseitigen Bereich nach links in der Breitenrichtung; und im zweiten Abschnitt besetzte Bereiche der Schweißpunkte auf einer rechten Seite der Durchdringungslochgruppe in der Breitenrichtung im linksseitigen Bereich größer sind mit Abstand von der Durchdringungslochgruppe im linksseitigen Bereich nach rechts in der Breitenrichtung, und
im dritten Abschnitt besetzte Bereiche der Schweißpunkte auf einer linken Seite der Durchdringungslochgruppe in der Breitenrichtung im rechtsseitigen Bereich größer sind mit Abstand von der Durchdringungsgruppe im rechtsseitigen Bereich nach links in der Breitenrichtung; und im dritten Abschnitt besetzte Bereiche der Schweißpunkte auf einer rechten Seite der Durchdringungslochgruppe in der Breitenrichtung im rechtsseitigen Bereich größer sind mit Abstand von der Durchdringungslochgruppe im rechtsseitigen Bereich nach rechts in der Breitenrichtung.

11. Einwegwindel nach Anspruch 7, wobei,
außerhalb der Schweißpunkte im ersten Abschnitt die Schweißpunkte auf der linken Seite der Durchdringungslochgruppe in der Breitenrichtung im breitenmäßigen Zentralbereich jeweils eine Form haben, die in einer Länge von vorne nach hinten größer ist mit Abstand von der Durchdringungslochgruppe im breitenmäßigen Zentralbereich nach links in der Breitenrichtung; und außerhalb der Schweißpunkte im ersten Abschnitt die Schweißpunkte auf der rechten Seite der Durchdringungslochgruppe in Breitenrichtung im breitenmäßigen Zentralbereich jeweils eine Form haben, die in einer Länge von vorne nach hinten größer ist mit Abstand von der Durchdringungslochgruppe im breitenmäßigen Zentralbereich nach rechts in der Breitenrichtung;
außerhalb der Schweißpunkte im zweiten Abschnitt die Schweißpunkte auf der linken Seite der Durchdringungslochgruppe in der Breitenrichtung im linksseitigen Bereich jeweils eine Form haben, die größer ist in einer Länge von vorne nach hinten mit Abstand von der Durchdringungslochgruppe im linksseitigen Bereich nach links in der Breitenrichtung; und außerhalb der Schweißpunkte im zweiten Abschnitt die Schweißpunkte auf der rechten Seite der Durchdringungslochgruppe in der Breitenrichtung im linksseitigen Bereich jeweils eine Form haben, die größer ist in einer Länge von vorne nach hinten mit Abstand von der Durchdringungslochgruppe im linksseitigen Bereich nach rechts in der Breitenrichtung, und
außerhalb der Schweißpunkte im dritten Abschnitt die Schweißpunkte auf der linken Seite der Durchdringungslochgruppe in der Breitenrichtung im rechtsseitigen Bereich jeweils eine Form haben, die größer ist in einer Länge von vorne nach hinten mit Abstand der Durchdringungslochgruppe im rechtsseitigen Bereich nach links in der Breitenrichtung; und außerhalb der Schweißpunkte im dritten Abschnitt die Schweißpunkte auf der rechten Seite der Durchdringungslochgruppe in der Breitenrichtung im rechtsseitigen Bereich jeweils eine Form haben, die größer ist in einer Länge von vorne nach hinten mit Abstand von der Durchdringungslochgruppe im rechtsseitigen Bereich nach rechts in der Breitenrichtung.

12. Einwegwindel nach Anspruch 7, wobei
eine große Anzahl von Schweißpunkten des Zwischenbereichs vorgesehen sind in vorbestimmten Intervallen, um die Deckschicht an einem Element an der Unterseite davon zu fixieren, in den links- und rechtsseitigen Zwischenbereichen, die von einem Bereich der ersten bis dritten Abschnitte ausgenommen sind, und
Bereiche der Schweißpunkte des Zwischenbereichs größer sind als die Bereiche der Schweißpunkte in den ersten bis dritten Abschnitten.

13. Einwegwindel nach Anspruch 1, wobei sie ferner aufweist:
eine Zwischenschicht (40) zwischen der Deckschicht und dem absorbierenden Element, um die Unterseite der Deckschicht zu kontaktieren, wobei
die Zwischenschicht wellenartige Falten hat, die durch Fixieren elastischer Elemente (42) in einem gedehnten Zustand an einer Grundmaterialschicht (41) und einem anschließenden Zusammenziehen der Grundmaterialschicht durch Kontraktion der elastischen Elemente gebildet sind.

14. Einwegwindel nach Anspruch 13, wobei die Zwischenschicht gebildet ist durch Zusammenhängen oder Kleben von zwei nicht verwebten Stoffen mit einer Feinheit von 2,0 bis 5,0 dtex, einem Faserbasisgewicht von 10 bis 30 g/m2 und einer Stärke von 01, bis 4 mm; Fixieren einer Vielzahl von länglichen nachgiebigen und elastischen Elementen mit einer Feinheit von 150 bis 1.240 dtex parallel zueinander in einem 100 bis 300 % gedehnten Zustand und in Intervallen von 3 bis 15 mm zwischen den vorangehenden nicht verwebten Stoffen; und Zusammenziehen der Grundmaterialschicht durch Kontraktion der elastischen Elemente, wodurch wellenartige Falten auf einer Oberfläche der Grundmaterialschicht in einer Richtung gebildet werden, die sich mit den elastischen Elementen kreuzt.

15. Einwegwindel nach Anspruch 14, wobei die Falten in einer Breitenrichtung der Windel gebildet sind.

16. Einwegwindel nach Anspruch 1, wobei
eine Zwischenschicht (40) zwischen der Deckschicht und dem absorbierenden Element eingefügt ist, um eine Unterseite der Deckschicht zu kontaktieren,
die Zwischenschicht gebildet ist durch Fixieren elastischer Elemente (42) in einem gedehnten Zustand an einer Grundmaterialschicht (41) und Zusammenziehen der Grundmaterialschicht durch Kontraktion der elastischen Elemente, wodurch wellenartige Falten auf der Oberfläche der Grundmaterialschicht gebildet werden, und die Falten wenigstens teilweise auf der Unterseite der Durchdringungslöcher in der Deckschicht angeordnet sind.

17. Einwegwindel nach Anspruch 16, wobei die Zwischenschicht durch Zusammenhängen oder Kleben von zwei nicht verwebten Stoffen mit einer Feinheit von 2,0 bis 5,0 dtex, einem Faserbasisgewicht von 10 bis 30 g/m² und einer Stärke von 1 bis 4 mm gebildet ist; Fixieren einer Vielzahl von länglichen nachgiebigen und elastischen Elementen mit einer Feinheit von 150 bis 1.240 dtex parallel zueinander in einem 100 bis 300% gedehnten Zustand und in Intervallen von 3 bis 15 mm zwischen den vorangehenden nicht verwebten Stoffen; und Zusammenziehen der Grundmaterialschicht durch Kontraktion der elastischen Elemente, wodurch wellenartige Falten auf der Oberfläche der Grundmaterialschicht in einer Richtung gebildet werden, die sich mit den elastischen Elementen kreuzt.

18. Einwegwindel nach Anspruch 17, wobei die Falten in einer Breitenrichtung der Windel gebildet sind.

## Revendications

1. Une couche jetable, comprenant une partie absorbante (10) qui a une feuille supérieure perméable au liquide (30) constituant une surface faisant au corps d'un porteur, une feuille imperméable au liquide (11) située sur un côté extérieur, un élément absorbant (50) interposé entre la feuille supérieure perméable au liquide et la feuille imperméable au liquide, et qui s'étend d'un bord supérieur (F1) d'un côté ventral (F) en passant par une portion d'entrejambe (C) jusqu'à un bord supérieur du côté dorsal (B) et le long d'un centre dans la direction de la largeur, dans laquelle
la feuille supérieure (30) a une région centrale transversale (31) s'étendant d'avant en arrière en passant par une portion correspondant au pli interfessier ; des régions intermédiaires (32) gauche et droite qui sont situées sur les côtés de la région centrale transversale et qui s'étendent d'avant en arrière en passant par des portions correspondant aux sommets des régions interfessières gauche et droite et, respectivement ; des régions droite et gauche (33) qui s'étendent d'avant en arrière le long d'un côté gauche de la région intermédiaire gauche et d'un côté droit de la région intermédiaire droite, respectivement,
un grand nombre de trous (H) de perméabilité sont formés à intervalles prédéterminés dans la région centrale transversale au moins dans une section entière transversale dans une direction avant-arrière correspondant au pli interfessier ; dans la région gauche au moins dans une section entière transversale dans la direction avant-arrière correspondant à une section située sur un côté gauche du sommet de la région interfessière gauche dans la direction transversale ; et dans la région droite au moins dans une section entière transversale dans la direction avant-arrière correspondant à une section située sur un côté droit du sommet de la région interfessière droite dans la direction transversale, et aucun trou de perméabilité n'est formé dans la région intermédiaire gauche au moins dans une section correspondant au sommet de la région interfessière gauche et dans la région intermédiaire droite au moins dans une section correspondant au sommet de la région interfessière droite.

2. La couche jetable selon la revendication 1, dans laquelle
une largeur des régions droite et gauche est plus petite qu'une largeur de la région centrale transversale.

3. La couche jetable selon la revendication 1, dans laquelle
la feuille supérieure est formée par un tissu non tissé avec une finesse de fibres brutes de 1,0 à 3,0 dtex et un grammage de fibre de 10 à 30 g/m²,
les trous de perméabilité sont disposés suivant un motif tel que les lignes de trous de perméabilité avec les trous de perméabilité alignés à intervalles prédéterminés dans la direction avant-arrière, sont prévues à intervalles prédéterminés dans la direction transversale,
une surface ouverte des trous de perméabilité est de 0,8 à 180 mm², un intervalle transversal entre les lignes de trous de perméabilité est de 1 à 5 mm, et un intervalle avant-arrière entre les trous de perméabilité dans les lignes de trous de perméabilité est de 1 à 10 mm, dans chacune des régions centrale transversale et droite et gauche.

4. La couche jetable selon la revendication 3, dans laquelle
le trou de perméabilité a une forme ouverte allongée dans laquelle une longueur avant-arrière (HL) est de 4 à 15 mm et une longueur transversale (HW) est plus petite que la longueur avant-arrière.

5. La couche jetable selon la revendication 1, dans laquelle
les trous de perméabilité dans les régions droite et gauche ont chacun une longueur ouverte transversale de 2 à 7 mm, et les trous de perméabilité dans la région centrale transversale ont chacun une longueur ouverte transversale de 4 à 10 mm, et la longueur transversale des trous de perméabilité dans les régions droite et gauche est plus petite que la longueur transversale des trous de perméabilité dans région centrale transversale.

6. La couche jetable selon la revendication 1, dans laquelle
les trous de perméabilité dans la région centrale transversale ont chacun une surface ouverte de 5 à 40 mm², les trous de perméabilité dans les régions droite et gauche ont chacun une surface ouverte de 10 à 50 mm², et la surface ouverte de chacun des trous de perméabilité dans les régions droite et gauche est plus grande que la surface ouverte de chacun des trous de perméabilité dans la région centrale transversale.

7. La couche jetable selon la revendication 1, dans laquelle
la feuille supérieure a des régions gauche et droite latérales périphériques s'étendant d'avant en arrière le long d'un côté gauche de la région gauche et d'un côté droit de la région droite, respectivement,
aucun trou de perméabilité est formé dans les régions gauche et droite latérales périphériques, et
un grand nombre de points de soudure (M) sont prévus à intervalles prédéterminés pour fixer la feuille supérieure à un élément sur un côté inférieur de façon que, dans une première section de ± 10 mm ou moins dans la direction transversale depuis les deux bords latéraux du groupe de trous de perméabilité dans la direction transversale dans la région centrale transversale ; dans une deuxième section de ± 10 mm ou moins dans la direction transversale depuis les deux bords latéraux du groupe de trous de perméabilité dans la région gauche ; et dans une troisième section de ± 10 mm ou moins dans la direction transversale depuis les deux bords latéraux du groupe de trous de perméabilité dans la région droite, la feuille supérieure est fixée à l'élément sur un côté inférieur de cette manière sans utiliser de colle, et la région centrale transversale et les régions gauche et droite ne sont pas fixées à l'élément sur le côté inférieur à l'exclusion des première à troisième sections.

8. La couche jetable selon la revendication 7, dans laquelle
les trous de perméabilité sont formés en réalisant des fentes d'avant en arrière dans la feuille supérieure aux positions de formation des trous de perméabilité et en étirant la feuille supérieure dans la direction transversale de manière à étendre les fentes, et la feuille supérieure est collée, pendant que les fentes sont étirées de cette façon, à un élément sur le côté inférieur de la feuille supérieure, dans des régions proches des deux bords latéraux des groupes de trous de perméabilité dans la direction transversale.

9. La couche jetable selon la revendication 7, dans laquelle
la feuille supérieure est formée par un tissu non tissé avec une finesse de fibres brutes de 1,0 à 3,0 dtex et un grammage de fibre de 10 à 30 g/m²,
les trous de perméabilité sont disposés suivant un motif tel que les lignes de trous de perméabilité avec les trous de perméabilité alignés à intervalles prédéterminés dans la direction avant-arrière, sont prévues à intervalles prédéterminés dans la direction transversale,
les points de soudure sont disposés suivant un motif tel que les lignes de points de soudure avec les points de soudure alignés à intervalles prédéterminés dans la direction avant-arrière, sont prévues à intervalles prédéterminés dans la direction transversale,
une surface ouverte des trous de perméabilité est de 0,8 à 180 mm2, un intervalle transversal entre les lignes de trous de perméabilité est de 1 à 5 mm, et un intervalle avant-arrière entre les trous de perméabilité dans les lignes de trous de perméabilité est de 1 à 10 mm, dans chacune des régions centrale transversale et droite et gauche, et
une surface des points de soudure est de 0,7 à 40 mm2, un intervalle transversal entre les lignes de points de soudure est de 2 à 15 mm, et un intervalle avant-arrière entre les points de soudure dans les lignes de points de soudure est de 5 à 30 mm.

10. La couche jetable selon la revendication 7, dans laquelle
dans la première section, les surfaces occupées par les points de soudure sur le côté gauche du groupe de trous de perméabilité dans la direction transversale dans la région centrale transversale sont significativement plus grandes que celles occupées par le groupe de trous de perméabilité dans la région centrale transversale vers la gauche dans la direction transversale ; dans la première section, les surfaces occupées par les points de soudure sur le côté droit du groupe de trous de perméabilité dans la direction transversale dans la région centrale transversale sont significativement plus grandes que celles occupées par le groupe de trous de perméabilité dans la région centrale transversale vers la droite dans la direction transversale,
dans la deuxième section, les surfaces occupées par les points de soudure sur le côté gauche du groupe de trous de perméabilité dans la direction transversale dans la région gauche sont significativement plus grandes que celles occupées par le groupe de trous de perméabilité dans la région gauche vers la gauche dans la direction transversale ; et dans la deuxième section, les surfaces occupées par les points de soudure sur le côté droit du groupe de trous de perméabilité dans la direction transversale dans la région gauche sont significativement plus grandes que celles occupées par le groupe de trous de perméabilité dans la région gauche vers la droite dans la direction transversale, et
dans la troisième section, les surfaces occupées par les points de soudure sur le côté gauche du groupe de trous de perméabilité dans la direction transversale dans la région droite sont significativement plus grandes que celles occupées par le groupe de trous de perméabilité dans la région droite vers la gauche dans la direction transversale ; et dans la troisième section, les surfaces occupées par les points de soudure sur le côté droit du groupe de trous de perméabilité dans la direction transversale dans la région droite sont significativement plus grandes que celles occupées par le groupe de trous de perméabilité dans la région droite vers la droite dans la direction transversale.

11. La couche jetable selon la revendication 7, dans laquelle
hors des points de soudure dans la première section, les points de soudure sur le côté gauche du groupe de trous de perméabilité dans la direction transversale dans la région centrale transversale ont chacun une forme qui est significativement plus grande dans la longueur avant-arrière que celle du groupe de trous de perméabilité dans la région centrale transversale vers la gauche dans la direction transversale ; et hors des points de soudure dans la première section, les points de soudure sur le côté droit du groupe de trous de perméabilité dans la direction transversale dans la région centrale transversale ont chacun une forme qui est significativement plus grande dans la longueur avant-arrière que celle du groupe de trous de perméabilité dans la région centrale transversale vers la droite dans la direction transversale,
hors des points de soudure dans la deuxième section, les points de soudure sur le côté gauche du groupe de trous de perméabilité dans la direction transversale dans la région gauche ont chacun une forme qui est significativement plus grande dans la longueur avant-arrière que celle du groupe de trous de perméabilité dans la région gauche vers la gauche dans la direction transversale ; et hors des points de soudure dans la deuxième section, les points de soudure sur le côté droit du groupe de trous de perméabilité dans la direction transversale dans la région gauche ont chacun une forme qui est significativement plus grande dans la longueur avant-arrière que celle du groupe de trous de perméabilité dans la région gauche vers la droite dans la direction transversale, et
hors des points de soudure dans la troisième section, les points de soudure sur le côté gauche du groupe de trous de perméabilité dans la direction transversale dans la région droite ont chacun une forme qui est significativement plus grande dans la longueur avant-arrière que celle du groupe de trous de perméabilité dans la région droite vers la gauche dans la direction transversale ; et hors des points de soudure dans la troisième section, les points de soudure sur le côté droit du groupe de trous de perméabilité dans la direction transversale dans la région droite ont chacun une forme qui est significativement plus grande dans la longueur avant-arrière que celle du groupe de trous de perméabilité dans la région droite vers la droite dans la direction transversale.

12. La couche jetable selon la revendication 7, dans laquelle
un grand nombre de points de soudure de régions intermédiaires sont prévus à intervalles prédéterminés pour fixer la feuille supérieure à un élément sur un côté inférieur de cette façon, dans les régions intermédiaires gauche et droite à l'exclusion des première à troisième sections, et
les surfaces des points de soudure de régions intermédiaires sont plus grandes que les surfaces des points de soudure dans les première à troisième sections.

13. La couche jetable selon la revendication 1, comprenant en outre :
une feuille intercalaire (40) entre la feuille supérieure et l'élément absorbant de façon à venir au contact du côté inférieur de la feuille supérieure, dans laquelle
la feuille intercalaire a des plis ondulés formés en fixant des éléments élastiques (42) dans un état étiré à une feuille (41) d'un matériau de base, puis en contractant le matériau de base par la contraction des éléments élastiques.

14. La couche jetable selon la revendication 13, dans laquelle
la feuille intercalaire est formée en collant ensemble deux tissus non tissés avec une finesse de 2,0 à 5,0 dtex, un grammage de fibre de 10 à 30 g/m², et une épaisseur de 0,1 à 4,0 mm ; en fixant une pluralité d'éléments allongés résistants et élastiques avec une finesse de 150 à 1240 dtex en parallèle dans un état étiré de 100 à 300% et à des intervalles de 3 à 15 mm entre les tissus non tissés précités ; et en contractant la feuille de matériau de base par contraction des éléments élastiques, de façon à former des plis ondulés sur la surface de la feuille de matériau de base dans une direction sécante aux éléments élastiques.

15. La couche jetable selon la revendication 14, dans laquelle
les plis sont formés dans la direction de la largeur d'une couche.

16. La couche jetable selon la revendication 1, dans laquelle,
une feuille intercalaire (40) est interposée entre la feuille supérieure et l'élément absorbant de façon à venir au contact du côté inférieur de la feuille supérieure,
la feuille intercalaire est formée en fixant des éléments élastiques (42) dans un état étiré à une feuille (41) d'un matériau de base et en contractant la feuille de matériau de base par la contraction des éléments élastiques, de façon à former des plis ondulés sur la surface de la feuille de matériau de base, et les plis sont au moins partiellement situés sur le côté inférieur des trous de perméabilité dans la feuille supérieure.

17. La couche jetable selon la revendication 16, dans laquelle
la feuille intercalaire est formée en collant ensemble deux tissus non tissés avec une finesse de 2,0 à 5,0 dtex, un grammage de fibre de 10 à 30 g/m², et une épaisseur de 0,1 à 4,0 mm ; en fixant une pluralité d'éléments allongés résistants et élastiques avec une finesse de 150 à 1240 dtex en parallèle dans un état étiré de 100 à 300% et à des intervalles de 3 à 15 mm entre les tissus non tissés précités ; et en contractant la feuille de matériau de base par contraction des éléments élastiques, de façon à former des plis ondulés sur la surface de la feuille de matériau de base dans une direction sécante aux éléments élastiques.

18. La couche jetable selon la revendication 17, dans laquelle
les plis sont formés dans la direction de la largeur d'une couche.
